# EUROPEAN PATENT APPLICATION

(11) **EP 1 964 564 A1**
(43) Date of publication of application: **03.09.2008**
(21) Application number: 07008026.2
(22) Date of filing: 19.04.2007
(51) Int. Cl.: A61K 31/4468, A61K 47/26, A61K 9/00

(54) **Breakthrough Pain Management**

(30) Priority: 01.03.2007 US 892601 P
(71) Applicant: LAB International SRL, Christ Church (BB)
(72) Inventor: Jouhikainen, Taneli., 02730 Espoo (FI); Lankinen, Tapio., 20880 Turku (FI); Sandström, Kenneth., 20750 Turku (FI)
(74) Representative: Müller-Boré & Partner Patentanwälte

(57) **Abstract**

The present invention is directed to a powdered formulation comprising an analgesic, preferably fentanyl, for use in pulmonary inhalation administration for the rapid analgesic titration of pain, in particular breakthrough pain. Upon administration, the powdered formulation is able to provide a narrower titration range in patients suffering from pain, as well as effective analgesic amounts of fentanyl in a shorter time and at lower dose levels of administered fentanyl when compared to fentanyl administered by an oral transmucosal route.

## Description

The present invention relates to powdered pharmaceutical compositions comprising an analgesic, for example an opioid such as fentanyl, for pulmonary inhalation administration. The pharmaceutical formulations of the present disclosure provide rapid titration efficacy in a narrow titration range, and are well-suited for the treatment and alleviation of pain in human and animal patients, for example acute pain, breakthrough pain or chronic pain, particularly in patients who are increasingly tolerant to opioid analgesics. The present disclosure further relates to methods for preparing the powdered pharmaceutical compositions, as well as a pharmaceutical kit comprising an inhalation device containing one or a plurality of dosage units of the powdered pharmaceutical formulation for the treatment of pain.

Persistent pain such as pain related to cancer can have a severe impact on a patient's quality of life, often interfering with the ability to eat, sleep, and interact with others. Patients in pain may be less willing to undergo treatment, less able to cope with side effects, and sometimes less willing to live. Control of pain is therefore an important and essential component of therapy, especially of cancer therapy. Mild pain can be treated with analgesic agents such as acetaminophen, NSAIDs and codeine, while persisting or increasing pain can be treated with weak or strong opioids. Moderate to severe chronic cancer pain can be treated with opioids where the therapeutic goal is to achieve maximal analgesia and minimize the occurrence of side effects, especially of adverse effects. While morphine is recommended as the opioid of first choice for the treatment of moderate to severe cancer pain, this recommendation is based largely on familiarity with morphine use and its availability and cost, not on proven clinical superiority over other opioids, which could provide significant and rapid analgesia at lower doses and without significant side effects if available in appropriate formulations and suitably administered. Alternative opioids and alternative routes of administration are needed to optimize the balance between analgesia and adverse events for individual patients. Tolerance build-up and the consequence of wide window of titration, with the titration not always successful, are problems commonly associated with opioid treatments. For instance, current transmucosal fentanyl treatments require a titration range of up to 8-fold of the starting dose to achieve successful analgesia in up to approximately 70% of the patients treated.

Fentanyl, N-phenyl-N-[1-(2-phenylethyl)-4-piperidyl] propanamide, (also known as N-(1-phenethyl-4-piperidyl) propionanilide dihydrogen, a prototype of the 4-anilidopiperidine class of compounds, is a potent opioid analgesic which interacts predominantly with opioid µ-receptors located in the brain and spinal cord (the central nervous system is the primary site of therapeutic action), and smooth muscle. Like morphine, fentanyl is a strong opioid agonist, and has been successfully used intravenously in context with general anesthetic regimens and for analgesia. Fentanyl functions to promote analgesia and sedation, and can increase a patient's tolerance for pain, as well as decrease the patient's perception of suffering.

Fentanyl is highly lipid soluble with a pKa of 8.4, and has been formulated in free base form for transdermal delivery in a patch device, such as the DURAGESIC® fentanyl transdermal system, which is indicated for treatment of chronic pain including pain associated with malignancy. DURAGESIC® is a rectangular transparent drug delivery patch device comprising a backing layer of polyester film, a drug reservoir of fentanyl and alcohol USP gelled with hydroxyethyl cellulose, an ethylene-vinyl acetate copolymer membrane that controls the rate of fentanyl delivery to a skin surface, a fentanyl-containing silicone adhesive, and a protective liner which is removed and discarded before application of the device to the skin. The amount of fentanyl released from a patch per hour is proportional to the surface area (25 µg/h per 10 cm²). In clinical practice, a patient is titrated to an analgesic dosage level that is a function of the patient's level of pain. The individual dosage level will also be a monotonic increasing function of a pre-existing opioid tolerance and of the pre-existing opioid's biochemical mode of action, such as acting as a pure agonist or mixed agonist-antagonist.

The recommended titration range for the DURAGESIC® fentanyl patch is from 12.5 µg/h to 100 µg/h, representing a titration window of 8-fold the lowest recommended dose. Serum fentanyl concentrations increase gradually following initial DURAGESIC® application, generally leveling off between 12 and 24 hours, and remaining relatively constant for the remainder of the 72 hour application period. Peak serum concentrations of fentanyl generally occur between 24 and 72 hours after initial application of the patch. DURAGESIC® 100 µg/h can provide analgesia approximately equivalent to 60 mg/day IM morphine in an acute pain model in non-opioid-tolerant patients. See Full Prescribing Information for DURAGESIC® (Fentanyl Transdermal System)(http://www.duragesic.com/active/janus/en_US/assets/common/company/pi/dura gesic.pdf, March 1, 2007).

Minimum effective analgesic serum concentrations of fentanyl in opioid-naive adult patients range from 0.2 to 1.2 ng/mL, with frequency of side effects increasing above serum levels of 2 ng/mL. Both the minimum effective concentration and the concentration at which toxicity occurs increase with tolerance. The rate and extent of development of tolerance varies widely among individuals treated for chronic pain. It is not possible to achieve rapid control of breakthrough pain with transdermal preparations because pain relief after dermal application is slow and plasma-steady-state is reached after 36 hours or more. About one-fifth and up to one-half of all patients on transdermal therapy need rescue dosing via another route, especially during dose titration and when the underlying cause of the pain or disease progresses. For patients with breakthrough pain that requires additional acute analgesic treatment to supplement the chronic pain treatment, analgesics are needed that can be titrated easily and rapidly, and provide relief from difficult-to-tolerate pain, preferably in an outpatient setting.

A pharmaceutical product that is available for the management of breakthrough cancer pain in patients with malignancies and who are already receiving and who are tolerant to opioid therapy for their underlying persistent cancer pain is Actiq®. Opioid-tolerant patients are those who are taking at least 60 mg morphine/day, 50 µg transdermal fentanyl/hour, or an equianalgesic dose of another opioid for a week or longer (reference: PDR, 2006 edition). Actiq® is intended for oral transmucosal absorption administration, and the active ingredient is fentanyl citrate, N-(1-phenethyl-4-piperidyl) propionanilide citrate (equivalent to 200, 400, 600, 800, 1200, or 1600 µg fentanyl base). In Actiq®, the fentanyl citrate resides in a solid matrix in a lollipop form, consisting of hydrated dextrates, citric acid, dibasic sodium phosphate, artificial berry flavor, magnesium stearate, modified food starch, and confectioner's sugar. Actiq® dissolves slowly in the mouth, and is hence cumbersome to use. In addition, the maximal plasma concentration is achieved as late as 20-70 minutes after application.

The oral transmucosal absorption of the fentanyl citrate lollipop provides an initial absorbed fraction of about 25% of administered dose from the buccal mucosa, and a more prolonged absorption fraction of the remaining 75% of the oral dose in the form of swallowed fentanyl which is absorbed from the gastrointestinal tract. Only approximately one-third of the swallowed dose becomes systemically available after intestinal and hepatic first pass metabolism. Both the blood fentanyl profile and the bioavailability of fentanyl will vary depending on the duration of lozenge consumption and on the fraction of the Actiq® dose that is absorbed through the oral mucosa versus the fraction that is swallowed. Such variations can be considerable if the lozenge is chewed and swallowed by the patient. In the management of breakthrough pain, an effective level using Actiq® is titrated using a dosage range from 200 µg to 1600 µg, representing an eight-fold difference in dose between the lowest and highest required dose. Despite this range, a substantial subset (up to or greater than 30%; see http://druglib.com/druginfo/actiq/pharmacology/, March 1, 2007) of the titrated patients may not experience significant analgesia (i.e., are not successfully titrated).

U.S. Publ. No. 20040034059 relates to a treatment to relieve acute pain with a relatively concentrated bolus composition of fentanyl citrate in a solvent using intranasal administration to give a faster onset of action than nasal titration of fentanyl, and a lower maximum plasma concentration comparable to intravenous administration. As disclosed in the application, administration of 3 mL of fentanyl citrate at a level of 500 microgram/mL (318 microgram/mL fentanyl base) via nebulization was effective in providing postoperative analgesia in 10 patients who underwent a variety of surgical procedures, but the duration of analgesia varied considerably from 5 to 90 minutes. Further, it was concluded that this route of administration is inefficient and labor intensive, and therefore generally is not recommended.

U.S. Patent Nos. 6,200,604 and 6,974,590 relate to a tablet formulation for buccal administration containing fentanyl citrate and a saliva activated, pH-adjusting effervescent agent of sodium carbonate, sodium bicarbonate and citric acid. This pharmaceutical product is commercially available as Fentora™. The Fentora™ label claim that the effervescent formulation enhances drug absorption through the buccal mucosa. But a known disadvantage of buccal formulations is the variations in rate of absorption by buccal versus swallowed oral administration. Also, the fraction swallowed and absorbed from the gastrointestinal tract is subject to first pass metabolism. In addition, as with Actiq®, Fentora™ has a broad titration range. Titration of Fentora™ is performed using a dose ranging from 100 mg to 800 µg. In addition, titration is obtained successfully in only approximately 70% of the patients within the eight-fold dose range of titration.

U.S. Patent No. 5,451,408 and U.S. Reissue No. 38,407 relate to an inhaled formulation of a partially liposome-encapsulated fentanyl which is claimed to offer a rapid onset of analgesia from absorption of unencapsulated or free opioid (in the range of 10-20% of the opioid dose), and a sustained analgesia from continued release of the liposome-encapsulated opioid. A potential disadvantage of this liposomal formulation is the required use of membrane-forming lipids in liquid suspension, which may be difficult to control in an industrial scale. Furthermore, this formulation does not enable immediate absorption of the drug, and hence the peak plasma concentrations will be obtained more gradually, generally within 20 minutes from start of inhalation.

U.S. Patent No. 6,264,981 relates to an oral transmucosal drug dosage using a solid solution containing a dissolution agent, which dosage has a shorter onset time than oral delivery. But once again, variations in the rate of absorption by buccal versus swallowed oral administration are expected. Also, the fraction swallowed and absorbed from the gastrointestinal tract is subject to first pass metabolism.

Farr et al., Adv Drug Delivery Rev. 58(9-10):1076-1088 (2006), review pulmonary delivery of opioids as pain therapeutics, as well as certain pharmacokinetics and pharmacodynamics of inhaled opioids, efficacy of inhaled opiates in comparison to injection, and other opioid dosage forms available for pain therapy. Farr et al. reported that peak plasma concentration may be achieved very rapidly after pulmonary absorption, in a manner that resembles intravenous infusion. But the plasma concentration of fentanyl administered via this route rapidly decreases, resulting in a short duration of efficacy.

Peng et al., Anesthesiology 90(2):576-599 (1999), review the use of fentanyl analgesia in the management of acute pain in adults. Peng et al. reported that aerosol transpulmonary administration of medication produces rapid, effective drug delivery as a result of the thin alveolar-blood barrier, high tissue perfusion, and enormous surface area of the lungs. Peng et al. indicate that while fentanyl can produce postoperative analgesia if administered as a nebulized aerosol, a liposome-encapsulated drug carrier system for fentanyl should be utilized to provide a controlled, sustained release system, which will overcome fentanyl's short duration of action (which Peng et al. assert is too brief for routine clinical use). But as described above, the liposome-encapsulated, nebuliser-aerosolized fentanyl formulations will not produce a rapid peak concentration of fentanyl.

Hung et al., Anesthesiology 83(2):277-284 (1995), report the pharmacokinetics of inhaled liposome-encapsulated fentanyl, and suggest that inhalation of a mixture of free and liposome-encapsulated fentanyl can provide a rapid increase and sustained plasma fentanyl concentrations. But controlling the ratio of free to liposome-encapsulated fentanyl in a reproducible and predictable manner is technically difficult. Mather et al., Br. J. Clin Pharmacology 46:37-43 (1998), report a pharmacokinetic analysis of systemic delivery by SmartMist™, which is a pulmonary administered form of aerosolized fentanyl. Mather et al. report that plasma concentrations were similar to those of intravenous injection. The time-averaged bioavailability of the fentanyl was >50% within 5 minutes of delivery.

Alexza Inc. recently reported an aerosolized formulation of fentanyl which produces a rapid peak concentration in plasma, similar to that of an intravenous infusion of fentanyl. Specifically, the Alexza system is a hand-held, electrically-heated, multiple-dose inhaler designed to generate and deliver excipient-free fentanyl aerosol for deep lung delivery. The fentanyl product consists of a disposable dose cartridge containing 25 doses each of 25 µg fentanyl, which is inserted into a reusable controller. The unformulated fentanyl from the Alexza system is vaporized into a condensation aerosol, and relies on a heat source that includes an inert metal substrate, a thin film of pure, unformulated fentanyl coated on the substrate, and an airway through which the patient inhales. The heated substrate reaches peak temperature after the patient starts to inhale. Next, the thin drug film is vaporized and as the drug vapor cools and condenses into pure drug aerosol particles drawn into the patient's lungs. This system has the disadvantage of being very complex and requires the use of heat to vaporize the drug. Alexza claims that this formulation is equivalent to an intravenous infusion with respect to both maximum concentration, and area under the time-concentration curve (AUC), indicating both an increase and rapid decrease of the fentanyl plasma concentration. No clinical outcome in terms of analgesia and dose-titration success was described or reported.

While fentanyl transpulmonary delivery appears to be an attractive administration route for the treatment of pain, a formulation for pulmonary inhalation administration is needed that addresses the lack of effective, rapidly-available, sufficiently long-acting, controlled and reproducible titrated delivery of fentanyl. Prior attempts to design and produce such a formulation have resulted in formulations that either have rapid absorption similar to that of intravenous infusions, or have slow absorption where peak concentrations appear 10 minutes or later after administration.

The present disclosure relates to powdered pharmaceutical compositions of an analgesic for pulmonary inhalation administration and use in the treatment and alleviation of pain, especially, of acute, chronic, or breakthrough pain in human or animal patients, for example breakthrough pain in cancer patients. The dose sparing effect of the presently disclosure compositions are particularly beneficial for administering drugs with known and potentially severe side effect, such as opioids. In a preferred embodiment, the active agent administered in the pharmaceutical composition is fentanyl or a pharmaceutically acceptable salt, derivative, or analog thereof, for example fentanyl citrate. The powdered formulation as presently disclosed, upon pulmonary inhalation, provides a unique combination of pharmacokinetic and clinical analgesic properties, producing rapid acting, long acting, reproducible and effective dose of the administered analgesic to patients experiencing breakthrough pain. In addition, the effective dose is readily titrated to address individual patient pain level symptoms. The effective dose of the analgesic is surprisingly lower when the powdered formulation is administered through inhalation when compared to other delivery routes, such as oral transmucosal delivery. For example, the dose of a powdered formulation comprising fentanyl is titrated to an effective dose in a narrower range and more successfully than with higher doses of fentanyl which are otherwise titrated and administered to patients in need thereof by means of a lozenge or buccal tablet fentanyl delivery system, or by means of other kind of inhaled or intranasal fentanyl delivery.

The present disclosure also relates to a pharmaceutical kit comprising a plurality of unit treatment dosage forms of the powdered pharmaceutical compositions disclosed herein, each of which is suitable for pulmonary inhalation or pulmonary administration together with an inhalation device, which kit is useful for treatment of chronic pain, acute pain or breakthrough pain, especially breakthrough pain in cancer patients. A preferred device useful to administer by pulmonary inhalation a powdered composition of the present disclosure is the TAIFUN® inhalator. The TAIFUN® inhalator, when used to administer a powdered composition of the present disclosure comprising fentanyl, or a pharmaceutically acceptable salt, derivative, or analog thereof, is sometimes referred to herein as Fentanyl TAIFUN®. Treatment of a patient suffering from breakthrough pain with fentanyl which is formulated according to the present disclosure and administered in the form of Fentanyl TAIFUN® provides rapid, easy and accurate control of cancer pain and cancer breakthrough pain through pulmonary inhalation of such fentanyl. In a preferred embodiment, Fentanyl TAIFUN® is used as a sole treatment of a patient in pain, for example chronic, acute, or most preferably, breakthrough pain.

One clinical indication for Fentanyl TAIFUN® is breakthrough cancer pain in patients with ongoing oral/parenteral/transdermal opioid therapy. Breakthrough cancer pain is a common problem during cancer therapy, especially during analgesic dose titration and when the disease progresses. Administration by pulmonary inhalation is easy and has been shown to produce an analgesic plasma concentration in only a few minutes. The action is faster than with transmucosal application. Pulmonary administration is a convenient way to mimic intravenous administration in a non-invasive way: rapid onset of pharmacological action; first pass hepatic metabolism is avoided; and nausea or vomiting does not prevent dosing as can happen with oral/transmucosal preparations.

The present disclosure is directed to methods for the preparation of a physically and chemically stable, homogenous powdered pharmaceutical composition for pulmonary administration comprising (1) particles comprising an analgesic, and (2) particles comprising a pharmaceutically acceptable carrier for pulmonary inhalation, the method comprising:
(a) suspending the particles comprising the analgesic and the pharmaceutically acceptable carrier in a suspending solvent, thereby forming a suspension, wherein the particles are essentially insoluble, sparingly soluble, slightly soluble, or partially soluble in the suspending solvent, and
(b) removing the suspending solvent from the suspension of step (a) to obtain the pharmaceutical composition.

The disclosure is further directed to pharmaceutical compositions produced by the above methods. For example, a physically and chemically stable, homogenous powdered pharmaceutical composition for pulmonary administration comprising (1) particles comprising an analgesic, and (2) particles comprising a pharmaceutically acceptable carrier for pulmonary inhalation, produced by a method comprising the steps of:
(a) suspending the particles comprising the analgesic and the pharmaceutically acceptable carrier in a suspending solvent, thereby forming a suspension, wherein the particles are essentially insoluble, sparingly soluble, slightly soluble, or partially soluble in the suspending solvent, and
(b) removing the suspending solvent from the suspension of step (a) to obtain the pharmaceutical composition.

In preferred embodiments, the powdered pharmaceutical composition of the present disclosure is dry powdered pharmaceutical composition. In other embodiments of the present disclosure, the analgesic is an opioid, more preferably fentanyl or a pharmaceutically acceptable salt, derivative, or analog thereof, most preferably fentanyl citrate. Preferably the particles comprising the analgesic have a mean particle size of less than about 10 microns, more preferably between about 0.5 to about 6 microns, most preferably at least about 1 micron to less than about 5 microns. The pharmaceutically acceptable carrier for pulmonary inhalation may be any water-soluble, for example a conventional physiologically acceptable solid particulate carrier, including but not limited to lactose, glucose, or mannitol, and in preferred embodiments, lactose monohydrate. Preferably the particles comprising the pharmaceutically acceptable carrier have a mean particle size of less than about 1000 microns, more preferably between about 20 to about 500 microns, most preferably between about 70 to about 130 microns.

In some embodiments, the suspending solvent used to suspend the particles comprising the analgesic or the pharmaceutically acceptable carrier comprises two or more hydrocarbon solvents, more preferably a combination of a non-polar hydrocarbon and a semi-polar hydrocarbon. In certain embodiments, the semi-polar hydrocarbon has a dielectric constant of equal to or greater than about 10 and less than about 50. In other embodiments, the suspending solvent comprises a combination of a hydrocarbon capable of forming an azeotrope with water and a second hydrocarbon. The hydrocarbon capable of forming an azeotrope with water may be selected from the group consisting of pentane, hexane, heptane, and combinations thereof. In preferred embodiment, the suspending solvent comprises n-hexane and an alcohol, such as 2-butanol. The suspending solvent may be removed from the suspension using methods well known to those of skill in the art, for example evaporation, filtration, spray drying, centrifugation, or any combination thereof. In addition, the suspended particles may be triturated and deagglomerated by using ultrasound, mechanical agitation, or a combination thereof.

In certain embodiments of the present disclosure, the pharmaceutical composition of the present disclosure has a fine particle fraction of between about 10% and about 60%, more preferably about 15% and about 35%, more preferably between about 18% and about 33%, and most preferably about 22% to about 28%. As used herein, fine particle fraction is defined as about the proportion of the particles comprising the analgesic that are less than about 5.8 microns when measured using a cascade impactor, or a corresponding method of analysis well known to those of skill in the art. Another embodiment of the present disclosure is inhalation device comprising the pharmaceutical compositions disclosed herein, wherein the inhalation device is designed to repeatedly meter a single dose quantity of the composition. Preferably, the inhalation device comprises the pharmaceutical composition in a single dose quantity, wherein the single dose quantity comprises 100 µg, 200 µg, 400 µg, or 800 µg of the analgesic.

A preferred embodiment of the present disclosure is a method for the preparation of a physically and chemically stable, homogenous powdered pharmaceutical composition for pulmonary administration comprising (1) particles comprising fentanyl or a pharmaceutically acceptable salt, derivative, or analog thereof, and (2) particles comprising a pharmaceutically acceptable carrier for pulmonary inhalation, the method comprising:
(a) suspending the particles comprising fentanyl or a pharmaceutically acceptable salt, derivative, or analog thereof, and the pharmaceutically acceptable carrier in a suspending solvent, thereby forming a suspension, wherein the particles are essentially insoluble, sparingly soluble, slightly soluble, or partially soluble in the suspending solvent, and
(b) removing the suspending solvent from the suspension of step (a) to obtain the pharmaceutical composition.

Another preferred embodiment of the present disclosure is a physically and chemically stable, homogenous powdered pharmaceutical composition for pulmonary administration comprising (1) particles comprising fentanyl or a pharmaceutically acceptable salt, derivative, or analog thereof, and (2) particles comprising a pharmaceutically acceptable carrier for pulmonary inhalation, produced by a method comprising the steps of:
(a) suspending the particles comprising fentanyl or a pharmaceutically acceptable salt, derivative, or analog thereof, and the pharmaceutically acceptable carrier in a suspending solvent, thereby forming a suspension, wherein the particles are essentially insoluble, sparingly soluble, slightly soluble, or partially soluble in the suspending solvent, and
(b) removing the suspending solvent from the suspension of step (a) to obtain the pharmaceutical composition.

In preferred embodiments, the powdered pharmaceutical composition comprising fentanyl or a pharmaceutically acceptable salt, derivative, or analog thereof is dry powdered pharmaceutical composition. Preferably, the pharmaceutically acceptable salt of fentanyl is fentanyl citrate. In other preferred embodiments, the particles comprising fentanyl or a pharmaceutically acceptable salt, derivative, or analog thereof have a mean particle size of less than about 10 microns, more preferably between about 0.5 to about 6 microns, most preferably at least about 1 micron to less than about 5 microns. The pharmaceutically acceptable carrier for pulmonary inhalation may be any water-soluble, for example a conventional physiologically acceptable solid particulate carrier, including but not limited to lactose, glucose, or mannitol, and in preferred embodiments, lactose monohydrate. Preferably the particles comprising the pharmaceutically acceptable carrier have a mean particle size of less than about 1000 microns, more preferably between about 20 to about 500 microns, most preferably between about 70 to about 130 microns.

In some embodiments, the suspending solvent used to suspend the particles comprising the fentanyl or a pharmaceutically acceptable salt, derivative, or analog thereof or the pharmaceutically acceptable carrier comprises two or more hydrocarbon solvents, more preferably a combination of a non-polar hydrocarbon and a semi-polar hydrocarbon. In certain embodiments, the semi-polar hydrocarbon has a dielectric constant of equal to or greater than about 10 and less than about 50. In other embodiments, the suspending solvent comprises a combination of a hydrocarbon capable of forming an azeotrope with water and a second hydrocarbon. The hydrocarbon capable of forming an azeotrope with water may be selected from the group consisting of pentane, hexane, heptane, and combinations thereof. In preferred embodiment, the suspending solvent comprises n-hexane and an alcohol, such as 2-butanol. The suspending solvent may be removed from the suspension using methods well known to those of skill in the art, for example evaporation, filtration, spray drying, centrifugation, or any combination thereof. In addition, the suspended particles may be triturated and deagglomerated by using ultrasound, mechanical agitation, or a combination thereof.

In certain embodiments of the present disclosure, the pharmaceutical composition comprising fentanyl or a pharmaceutically acceptable salt, derivative, or analog thereof has a fine particle fraction of between about 10% and about 60%, more preferably about 15% and about 35%, more preferably about 18% and about 33%, and most preferably about 22% to about 28%. As used herein, fine particle fraction is defined as about the proportion of the particles comprising fentanyl or a pharmaceutically acceptable salt, derivative, or analog thereof, for example fentanyl citrate, that are less than about 5.8 microns when measured using a cascade impactor, or a corresponding method of analysis well known to those of skill in the art. Another embodiment of the present disclosure is a physically and chemically stable, homogenous powdered pharmaceutical composition for pulmonary administration comprising fentanyl or a pharmaceutically acceptable salt, derivative, or analog thereof, and a pharmaceutically acceptable carrier for pulmonary inhalation, wherein the composition has a fine particle fraction of between about 10 and about 60%, more preferably about 15% and about 35%, more preferably about 20% and about 30%, most preferably about 22 and about 28%. Another embodiment of the present disclosure is inhalation device comprising the pharmaceutical compositions disclosed herein, wherein the inhalation device is designed to repeatedly meter a single dose quantity of the composition. Preferably, the inhalation device comprises the pharmaceutical composition in a single dose quantity, wherein the single dose quantity comprises 100 µg, 200 µg, 400 µg, or 800 µg of fentanyl or a pharmaceutically acceptable salt, derivative, or analog thereof.

Yet another preferred embodiment of the present disclosure is a method for the preparation of a physically and chemically stable, homogenous powdered pharmaceutical composition for pulmonary administration comprising (1) particles comprising fentanyl or a pharmaceutically acceptable salt, derivative, or analog thereof, and (2) particles comprising lactose monohydrate, the method comprising:
(a) suspending the particles comprising fentanyl or a pharmaceutically acceptable salt, derivative, or analog thereof, and lactose monohydrate in a suspending solvent, wherein the suspending solvent comprises n-hexane and 2-butanol, thereby forming a suspension, and
(b) removing the suspending solvent from the suspension of step (a) to obtain the pharmaceutical composition.

Still another preferred embodiment of the present disclosure is physically and chemically stable, homogenous powdered pharmaceutical composition for pulmonary administration comprising (1) particles comprising fentanyl or a pharmaceutically acceptable salt, derivative, or analog thereof, and (2) particles comprising lactose monohydrate, produced by a method comprising the steps of:
(a) suspending the particles comprising fentanyl or a pharmaceutically acceptable salt, derivative, or analog thereof, and lactose monohydrate in a suspending solvent, wherein the suspending solvent comprises n-hexane and 2-butanol, thereby forming a suspension, and
(b) removing the suspending solvent from the suspension of step (a) to obtain the pharmaceutical composition.

Other embodiments of the present disclosure are directed to a physically and chemically stable, homogenous powdered pharmaceutical composition for pulmonary administration to human patients, comprising from about 100 µg to about 800 µg of fentanyl or a pharmaceutically acceptable salt, derivative, or analog thereof, and a pharmaceutically acceptable carrier, said composition providing a mean peak concentration of fentanyl from a mean of about 30 seconds to about 10 minutes after administration, more preferably from about 30 seconds to about 3 minutes after administration, most preferably about 30 seconds to about 1 minute, and a concentration of fentanyl that does not decrease more than about 50%, 45%, 40%, 35%, 30%, 25%, 20%, 15%, or 10% between about 10 minutes and about 2 hours after administration. In other embodiments, the concentration of fentanyl that does not decrease more than about 50%, 45%, 40%, 35%, 30%, 25%, 20%, 15%, or 10% between about 10 minutes and about 1 hour after administration.

Another embodiment of the present disclosure is a method for treating pain in human patients in need of such treatment, comprising administering to a human patient by pulmonary administration a pharmaceutical composition comprising from about 100 µg to about 800 µg of fentanyl or a pharmaceutically acceptable salt thereof, said composition providing a mean peak concentration of fentanyl from a mean of about 30 seconds to 10 minutes after administration, more preferably from about 30 seconds to about 3 minutes after administration, most preferably about 30 seconds to about 1 minute, and a concentration of fentanyl that does not decrease more than about 50%, 45%, 40%, 35%, 30%, 25%, 20%, 15%, or 10% between about 10 minutes and about 2 hours after administration, wherein the composition provides pain relief to the patient in less than 10, 9, 8, 7, 6, 5, 4, 3, 2, or 1 minutes. In other embodiments, the concentration of fentanyl that does not decrease more than about 50%, 45%, 40%, 35%, 30%, 25%, 20%, 15%, or 10% between about 10 minutes and about 1 hour after administration.

Still another embodiment of the present disclosure is a method for treating pain in human patients in need of such treatment, comprising administering to a human patient by pulmonary administration a pharmaceutical composition comprising from about 100 µg to about 800 µg of fentanyl or a pharmaceutically acceptable salt thereof, said composition providing a mean peak concentration of fentanyl from a mean of about 30 seconds to 10 minutes after administration, more preferably from about 30 seconds to about 3 minutes after administration, most preferably about 30 seconds to about 1 minute, wherein the mean peak concentration is less than that of an equivalent dose of fentanyl administered intravenously, and a concentration of fentanyl that is greater between about 10 minutes and about 2 hours after administration than the mean minimum concentration of an equivalent dose of fentanyl administered intravenously between about 10 minutes and about 2 hours after administration, wherein the composition provides pain relief to the patient in less than 10, 9, 8, 7, 6, 5, 4, 3, 2, or 1 minutes. In other embodiments, the concentration of fentanyl that does not decrease more than about 50%, 45%, 40%, 35%, 30%, 25%, 20%, 15%, or 10% between about 10 minutes and about 1 hour after administration.

Yet another embodiment of the present disclosure is a method for titrating breakthrough pain in at least about 80% of the members of a patient population experiencing such breakthrough pain, comprising administering to said members in need of such treatment a pharmaceutical composition comprising from about 100 µg to about 400 µg of fentanyl or a pharmaceutically acceptable salt thereof by pulmonary administration, said composition providing a mean peak concentration of fentanyl from a mean of about 30 seconds to 10 minutes after administration, more preferably from about 30 seconds to about 3 minutes after administration, most preferably about 30 seconds to about 1 minute, and a concentration of fentanyl that does not decrease more than about 50%, 45%, 40%, 35%, 30%, 25%, 20%, 15%, or 10% between about 10 minutes and about 2 hours after administration, wherein the composition provides pain relief to the patient in less than about 10, 9, 8, 7, 6, 5, 4, 3, 2, or 1 minutes. In other embodiments, the concentration of fentanyl that does not decrease more than about 50%, 45%, 40%, 35%, 30%, 25%, 20%, 15%, or 10% between about 10 minutes and about 1 hour after administration.

Another embodiment of the present disclosure is a method of treating acute, chronic, or breakthrough pain in a patient in need thereof, comprising administering to the patient in need of such treatment a pharmaceutical composition comprising from about 100 µg to about 800 µg of an analgesic by pulmonary administration with an inhaler to obtain an essentially immediate release absorption of a respirable fraction of the analgesic, said composition providing a mean peak concentration of the analgesic from a mean of about 30 seconds to 10 minutes after administration, more preferably from about 30 seconds to about 3 minutes after administration, most preferably about 30 seconds to about 1 minute, and which is less than the mean maximum concentration of an intravenous injection of a similar total dose of the analgesic, combined with a more gradual absorption of another non-respirable fraction of the analgesic to obtain a prolonged effective concentration of the analgesic with a mean minimum concentration after at least one hour and up to two hours from the administration, and which is more than the mean minimum concentration of an intravenous injection of a similar total dose.

In a preferred embodiment, the powdered pharmaceutical composition is dry powdered pharmaceutical composition. In certain embodiments, the mean peak concentration of fentanyl is from about 250 pg/mL, 300 pg/mL, 350 pg/mL, 400 pg/mL, 450 pg/mL, or 500 pg/mL of fentanyl, to about 3000 pg/mL, 2750 pg/mL, 2500 pg/mL, 2250 pg/mL, 2000 pg/mL of fentanyl, or any ranges in that fall between the above values. In other embodiments, between about 10 minutes and about 2 hours after administration, the mean maximum plasma concentration of fentanyl is about 125 pg/mL, 150 pg/mL, 160 pg/mL, 170 pg/mL, 180 pg/mL, 190 pg/mL, 200 pg/mL, 210 pg/mL, 220 pg/mL, 230 pg/mL, 240 pg/mL, 250 pg/mL to about 2000 pg/mL, 1900 pg/mL, 1800 pg/mL, 1700 pg/mL, 1600 pg/mL, 1500 pg/mL, 1400 pg/mL, 1300 pg/mL, or 1200 pg/mL, or any ranges in that fall between the above values.

Other embodiments of the present disclosure are directed to methods of relieving by analgesia an episode of breakthrough pain in at least about 35%, 40%, 45%, 50%, 55%, 60%, 65%, 70%, 75%, 80%, 85%, 90%, 95%, or 96% of the members of a patient population experiencing such breakthrough pain, comprising administering to said members by pulmonary inhalation a 20 µg, 30 µg, 40 µg, 50 µg, 100 µg, 150 µg, 200 µg, 250 µg, 300 µg, 350 µg, 400 µg, 450 µg, 500 µg, 550 µg, 600 µg, 650 µg, 700 µg, 750 µg, or 800 µg dose, or any doses in that fall between the above doses, of any one of the pharmaceutical compositions disclosed herein. These doses may be administered as a single dose, or as multiple doses.

In another embodiment, the present disclosure is directed to a method for the preparation of a powdered pharmaceutical formulation comprising a triturate of fentanyl citrate and a pharmaceutically acceptable carrier for pulmonary inhalation use comprising forming a suspension of particles of fentanyl citrate having a mean particle size of about 1 to 5 microns and particles of a pharmaceutically acceptable carrier for pulmonary inhalation use having a mean particle size of about 70 to 130 microns in a suspending solvent in which the particles are sparingly soluble, consisting of a combination of a hydrocarbon suspending solvent and a second hydrocarbon suspending solvent, applying one or more external energy means to triturate and deagglomerate the suspended particles, and evaporating the suspending solvent to leave a powder suitable for pulmonary administration.

In yet another embodiment, the present disclosure is directed to a method for the preparation of a powdered pharmaceutical formulation comprising a triturate of fentanyl citrate and a pharmaceutically acceptable carrier for pulmonary inhalation use comprising suspending particles of fentanyl citrate having a mean particle size of 1 to 5 microns in a suspending solvent in which the particles are sparingly soluble, consisting of a combination of a hydrocarbon suspending solvent and a second hydrocarbon suspending solvent, applying one or more external energy means to triturate and deagglomerate the suspended particles, adding particles of a pharmaceutically acceptable carrier for pulmonary inhalation use having a mean particle size of about 1 to 5 microns to the suspended triturate, optionally applying additional external energy means, and evaporating the suspending solvent.

In other embodiments of the present disclosure, the powdered pharmaceutical formulation prepared as described herein can be contained in a multiple use inhalation device capable of repeatedly metering a single dose quantity of the formulation. In another aspect, the powdered pharmaceutical formulation prepared as described herein can be contained as a single dose quantity in a single use or multiple use inhalation device. Currently preferred single dose levels include a 100 µg dose of fentanyl and a 200 µg dose of fentanyl.

In still other embodiments of the present disclosure, a 100 µg dose of fentanyl of the formulation of fentanyl as described herein when administered by pulmonary inhalation can provide fentanyl in a pharmacokinetic profile comprising a geometric LS mean Cmax of 362 pg/ml, a median Tmax of 0.034 hours, a geometric LS mean AUCT of 1487 pg.h/ml, a geometric LS mean AUCI of 1839 pg.h/ml, a geometric LS mean AUC 0-20 (AUC 0 minutes to 20 minutes) of 58 pg.h/ml, a geometric LS mean AUC 0-30 (AUC 0 minutes to 30 minutes) of 86 pg.h/ml, a geometric LS mean AUC 20-I (AUC 20 minutes to infinity) of 1761 pg.h/ml, and a geometric LS mean AUC 30-I (AUC 30 minutes to infinity) of 1729 pg.h/ml.

In another aspect, a 200 µg dose of fentanyl of the formulation of fentanyl as described herein when administered by pulmonary inhalation can provide fentanyl in a pharmacokinetic profile comprising a geometric LS mean Cmax of 945 pg/ml, a median Tmax of 0.0167 hours, a geometric LS mean AUCT of 3435 pg.h/ml, a geometric LS mean AUCI of 3714 pg.h/ml, a geometric LS mean AUC 0-20 (AUC 0 minutes to 20 minutes) of 139 pg.h/ml, a geometric LS mean AUC 0-30 (AUC 0 minutes to 30 minutes) of 201 pg.h/ml, a geometric LS mean AUC 20-I (AUC 20 minutes to infinity) of 3559 pg.h/ml, and a geometric LS mean AUC 30-I (AUC 30 minutes to infinity) of 3494 pg.h/ml.

The following drawings form part of the present specification and are included to further demonstrate certain aspects of the present invention. The invention may be better understood by reference to one or more of these drawings in combination with the detailed description of specific embodiments presented herein.

Figure 1 shows Mean Plasma Fentanyl Concentrations (pg/ml) following the administration of the reference (Actiq® 200µg) and study drugs (TAIFUN^{®} 100µg, 200µg, 400µg and 800µg).

Figure 2 shows Mean Plasma Fentanyl Concentrations (pg/ml) following the administration of the reference (Actiq^{®} 200µg) and study drugs (TAIFUN^{®} 100µg, 200µg, 400µg and 800µg) - Semi-Log Scale.

Figure 3 shows Mean Plasma Fentanyl Concentrations (pg/ml) following the administration of the reference (Actiq^{®} 200µg) and study drugs (TAIFUN^{®} 100µg, 200µg, 400µg and 800µg) for 0-30 minutes (0.5 hours) - linear scale.

Figure 4 shows Mean Plasma Fentanyl Concentrations (pg/ml) following the administration of the reference (Actiq^{®} 200µg) and study drugs (TAIFUN^{®} 100µg, 200µg, 400µg and 800µg) for 0-30 minutes (0.5 hours) - Semi-Log Scale.

Figure 5 shows Mean Plasma Fentanyl Concentrations (pg/ml) following the administration of the reference (Actiq 200µg) and study drugs (TAIFUN^{®} 100µg, 200µg, 400µg and 800µg) for 0-240 minutes (4 hours) - linear Scale.

Figure 6 shows Mean Plasma Fentanyl Concentrations (pg/ml) following the administration of the reference (Actiq® 200µg) and study drugs (TAIFUN^{®} 100µg, 200µg, 400µg and 800µg) for 0-240 minutes (4 hours) - Semi-Log Scale.

Figure 7 is a graph showing the individual pharmacokinetic parameters for Cmax (pg/ml).

Figure 8 is a graph showing the individual pharmacokinetic parameters for AUCT (pg.h/ml).

The present disclosure relates to a powdered formulation of an analgesic capable of delivering a therapeutically effective amount for treating pain, such as cancer pain, in a human or animal patient through pulmonary or inhalation administration. Preferably the analgesic is an opioid, more preferably fentanyl, or a salt, analog, or derivative thereof. In preferred embodiments, the powdered formulation is a dry-powdered formulation that is physically stable, chemically stable, and substantially homogenous. The dry-powdered formulation preferably has a moisture content such that the particles are readily dispersible in an inhalation device to form an aerosol of the powdered formulation containing micronized fentanyl prepared by the methods described in this disclosure. This moisture content is generally below about 10% by weight (% w/w) water, usually below about 5% w/w water, and preferably less than about 3% w/w water. A therapeutically effective amount of the analgesic is the amount present in the powdered formulation that is needed to provide the desired level of active agent in the subject to be treated to give the anticipated physiological response.

The powdered formulations of the present disclosure, when administered through the pulmonary or inhalation route, preferably provide a unique combination of the desirable properties of both intravenous injection and transmucosal oral preparations of analgesics, without the disadvantages often associated with these routes of delivery. For example, the powdered formulations of the present disclosure may produce a very rapid absorption with early and high bioavailability of the analgesic (such as with intravenous injection or some conventional inhalation solutions), as well as a prolonged effective drug concentration of the analgesic (such as with transmucosal oral preparations), but without the general, undesired properties associated with intravenous and transmucosal routes of administration, e.g., rapid decrease of the concentration of the active agent with short analgesic action (such as with intravenous injection, or some conventional inhalation solutions), or slow absorption of the active agent with a slow onset of action (such as with transmucosal oral preparations). Further, the preferred formulations of the present disclosure provide rapid titration efficacy in a narrow titration range.

The pharmacokinetics of the powdered formulations of the present disclosure are well-suited for the treatment and alleviation of pain in human and animal patients, such as acute pain, breakthrough pain, and chronic pain. The powdered formulations may be particularly well-suited for the management of cancer pain and cancer breakthrough pain, particularly in patients with malignancies. The pulmonary powdered formulations further allow for the management and treatment of breakthrough, pain in a higher percentage of patients at a lower dosage, when compared to other administration routes such as intravenous and oral transmucosal. Given the potential side effects of analgesics such as opioids, therapeutic treatment with reduced doses is a significant and highly desired benefit. The powdered formulations for inhalation also may be administered to patients who are increasingly tolerant to opioid analgesics.

The powdered formulations disclosed herein can allow for maximal plasma concentrations to be achieved within seconds or minutes after administration. The maximal plasma concentration is achieved within about 10 minutes of pulmonary or inhalation administration of the powdered formulation of the analgesic. In preferred embodiments, maximal plasma concentration is achieved in less than about 10, 9.5, 9.0, 8.5, 8.0, 7.5, 7.0, 6.5, 6.0, 5.5, 5.0, 4.5, 4.0, 3.5, 3.0, 2.5, 2.0, 1.5, 1.0, or 0.5 minutes. More preferably, the maximal plasma concentration is achieved in about 4.0, 3.75, 3.5, 3.25, 3.0, 2.75, 2.5, 2.25, or 1.0 minutes, or in about 55, 50, 45, 40, 35, 30, 25, 20, 15, 10, 9, 8, 7, 6, 5, 4, 3, 2, or 1 seconds. In another embodiment, the powdered formulation is able to provide analgesic relief to a patient suffering from breakthrough pain with a mean maximum plasma concentration of the analgesic(s) of from about 300 pg/mm to about 3000 pg/mL of the analgesic(s) in a mean time of about 30 seconds to about 10 minutes after administration, and a mean maximum plasma concentration of about 100 pg/mm to about 2000 pg/mL between the time points of about 10 minutes to about 2 hours after administration. In yet another embodiment, the powdered formulation is able to provide analgesic relief to a patient suffering from breakthrough pain with a mean maximum plasma concentration of the analgesic(s) of from about 200 pg/mm to about 2000 pg/mL of the analgesic(s) in a mean time of about 30 seconds to about 10 minutes after administration, and a mean maximum plasma concentration of about 100 pg/mm to about 1000 pg/mL between the time points of about 10 minutes to about 2 hours after administration. Preferably, analgesic relieve is obtained through the pulmonary or inhalation administration of from about 100 µg to about 800 µg of the powdered formulation, more preferably from about 100 µg to about 400 µg of the powdered formulation, and most preferably from about 100 µg to about 200 µg of the powdered formulation.

Surprisingly, the powdered formulations for pulmonary or inhalation administration of the present disclosure are able to relieve an episode of breakthrough pain by analgesia over a substantially narrow titration range (approximately four-fold) in greater than about 90% of patient. This is in sharp contrast to the approximately eight-fold range required for approximately 90% of the patients treated with opioid analgesics in general. The use of about 400 µg doses of dry-powder fentanyl formulations to control breakthrough pain in at least about 90% of patients is an example of the unique characteristics and pharmacokinetic profiles of the formulations disclosed herein. In a preferred embodiment of the present disclosure, pulmonary or inhalation administration of about 200 to about 400 µg of the powdered formulation disclosed herein provides a method for titrating breakthrough pain in up to about 85%, 86%, 87%, 88%, 89%, 90%, 91%, 92%, 93%, 94%, 95%, 96%, 97%, 98%, or 99% of the members of a patient population experiencing breakthrough pain, such as breakthrough cancer pain. In a more preferred embodiment of the present disclosure, pulmonary or inhalation administration of about 100 to about 400 µg of the fentanyl powdered formulation disclosed herein provides a method for titrating breakthrough pain in up to about 70%, 71%, 72%, 73%, 74%, 75%, 76%, 77%, 78%, 79%, 80%, 81%, 82%, 83%, 84%, 85%, 86%, 87%, 88%, 89%, 90%, 91 %, 92%, 93%, 94%, 95%, 96%, 97%, 98%, or 99% of the members of a patient population experiencing breakthrough pain, such as breakthrough cancer pain.

In another embodiment of the present disclosure, pulmonary or inhalation administration of about 100 to about 200 µg of the powdered formulation disclosed herein provides a method for titrating breakthrough pain in up to about 30%, 35%, 40%, 45%, 50%, 55%, 60%, 65%, 70%, 75%, 80%, 85%, 90%, 95%, 96%, 97%, 98%, or 99% of the members of a patient population experiencing breakthrough pain, such as breakthrough cancer pain. In a more preferred embodiment of the present disclosure, pulmonary or inhalation administration of about 100 µg to about 200 µg of the fentanyl powdered formulation disclosed herein provides a method for titrating breakthrough pain in up to about 30%, 35%, 39%, 40%, 45%, 50%, 55%, 60%, 65%, 70%, 74%, 75%, or 80% of the members of a patient population experiencing breakthrough pain, such as breakthrough cancer pain.

The present disclosure also relates to methods for preparing the powdered formulations disclosed herein. In a preferred method for preparing the formulation, the particles of an analgesic are suspended in a suspending solvent in which the particles are insoluble, sparingly soluble, slightly soluble, or partially soluble, and the suspending solvent is evaporated from the suspension, which generates a physically stable, homogeneous powder preparation. Physiologically acceptable additives such as a carrier may optionally be added to the suspension. In preferred embodiments, the powdered formulation comprises microparticles (in the desired particle size range) of the analgesic as an active agent in combination with one or more carriers suitable for pulmonary or inhalation administration. The carrier is preferably a water-soluble carrier, for example a conventional physiologically acceptable solid particulate carrier. The powdered formulations may also contain two or more active agents. Preferably, the microparticles of the formulation are distributed homogeneously in contact with or weakly bound to the surface of the carrier(s). The powdered formulations of the present disclosure are administered from a powder inhaler device, preferably a dry-powder metered dose inhalation device.

As described, the present disclosure relates to a powdered formulation of an analgesic capable of delivering an effective dose for treating pain, such as acute pain, breakthrough pain, or chronic pain. An analgesic (colloquially known as a painkiller) is any member of the diverse group of drugs used to relieve pain, i.e., achieve analgesia. Analgesic drugs act in various ways on the peripheral and central nervous system, and include but are not limited to paracetamol (acetaminophen), phenacetin, non-steriodal anti-inflammatory drugs (NSAIDs) (e.g., salicylates, metamizole, aspirin (acetylsalicylic acid), ibuprofen, indomethacin, diclofenac sodium, diflunisal, ethenzamide), and synthetic drugs with narcotic properties (e.g., tramadol).

A preferred class of analgesics for formulation according to the present disclosure are narcotic analgesics, in particular opioids. Opioids are associated with a range of adverse drug reactions, and the powdered formulations of the present disclosure minimize these adverse reactions. A preferred opioid for the powdered formulations of the present disclosure is fentanyl, as well as salts, analogs, and derivatives thereof. Fentanyl can be formulated as a free base, or more preferably in the form of a pharmaceutically acceptable salt such as a citrate salt, e.g., fentanyl citrate or N-(1-phenethyl-4-piperidyl) propionanilide citrate (1:1). Fentanyl analogs include but are not limited to alfentanil, sufentanil, remifentanil, and carfentanil. Methods for synthesizing fentanyl, as well as salts, analogs, and derivatives thereof, are well known to those of skill in the art.

Narcotic analgesics which may be suitable for formulation according to the present disclosure for pulmonary or inhalation administration include but are not limited to Acetorphine, Acetyldihydrocodeine, Acetyldihydrocodeinone, Acetylmorphone, Alfentanil, Allylprodine, Alphaprodine, Anileridine, Bemidone, Benzylmorphine, beta-4-morpholinylethylmorphine, Betaprodine, Betmeprodine, Bezitramide, Buprenorphine, Butorphanol, Carfentanil, Clonitazene, Codeine, Codeine Methyl Bromide, Codeine-N-Oxide, Codeine Phosphate, Codeine Sulfate, Codeinone, Cyclazocine, Cyclorphan, Desomorphine, Dextromoramide, Dextropropoxyphene, Dezocine, Diacetyldihydrocodeine, Diacetyldihydromorphine, Diamorphine, Diampromide, Diethylthiambutene, Difenoxin, Dihydrocodeine, Dihydrocodeinone Enol Acetate, Dihydrohydroxycodeinone, Dihydrodesoxymorphine. Dihydroetorphine, Dihydroheroin, Dihydroisocodeine, Dihydromorphine, Dimenoxadol, Dimepheptanol, Dimethylthiambutene, Dioxaphetyl Butyrate, Diphenoxylate, Dipipanone, Diprenorphine, Dipropanoylmorphine, Dromoran, Ethylketocyclazocine, Eptazocine, Ethoheptazine, Ethylmethlythiambutene, Ethylmorphine, Etonitazene, Etorphine, Fentanyl, Diamorphine (Heroin), Hydrocodone, Hydrocodone Bitartrate, Hydromorphone, Hydroxypethidine, Isomethadone, Ketobemidone, Laudanum, Lefetamine, Levallorphan, Levo-alphacetylmethadol, Levomethorphan, Levomethadone, Levopropoxyphene, Levorphanol, Lofentanil, Loperamide, Methadone, Meperidine, Meptazinol, Metazocine, Methadone Hydrochloride, Metopon, Monoacetylmorphine, Morphine, Morphine Derivatives (Morphine-N-Oxide, Morphine, Morphinone, Morphine-6-glucuronide), MPPP, Myrophine, Nalbuphine, Nalmefene, Nalorphine, Naloxone, Naltrexone, Narceine, Nicocodeine, Nicodicodeine, Nicomorphine, Norcodeine, N-allyl-normorphine, Norlevorphanol, Normethadone, Normorphine, Norpipanone, Ohmefentanyl, Omnopon, Opium, Oxycodone, Oxymorphone, Pantopon, Papaveretum, Paregoric, Pentazocine, Pethidine, Phenadoxone, Phenazocine, Pheoperidine, PEPAP, Pethidine (Meperidine), Phenoperidine, Pholcodeine, Piminodine, Piritramide, Prodine, Proheptazine, Promedol, Properidine, Propiram, Propoxyphene, Racemethorphan, Racemorphan, Remifentanil, Sufentanil, Tetrapon, Thebacon, Thebaine, Thiofentanil, Tilidine, Tramadol, and Trimeperidine, as well as salts, esters, analogs, and derivatives thereof, and any combination thereof.

Non-narcotic analgesics which may be suitable for formulation according to the present disclosure for pulmonary or inhalation administration include but are not limited to Acetaminophen, Acetaminosalol, Acetanilide, Acetylsalicylsalicylic Acid, Alclofenac, Alminoprofen, Aloxiprin, Aluminum Bis(acetylsalicylate), Aminochlorthenoxazin,2-Amino-4-picoline, Aminopropylon, Aminopyrine, Ammonium Salicylate, Antipyrine, Antipyrine Salicylate, Antrafenine, Apazone, Aspirin, Benorylate, Benoxaprofen, Benzpiperylon, Benzydamine, p-Bromoacetanilide, 5-Bromosalicylic Acid Acetate, Bucetin, Bufexamac, Bumadizon, Butacetin, Calcium Acetylsalicylate, Carbamazepine, Carbetidine, Carbiphene, Carsalam, Chloralantipyrine, Chlorthenoxazin(e), Choline Salicylate, Cinchophen, Ciramadol, Clometacin, Cropropamide, Crotethamide, Dexoxadrol, Difenamizole, Diflunisal, Dihydroxyaluminum Acetylsalicylate, Dipyrocetyl, Dipyrone, Emorfazone, Enfenamic Acid, Epirizole, Etersalate, Ethenzamide, Ethoxazene, Etodolac, Felbinac, Fenoprofen, Floctafenine, Flufenamic Acid, Fluoresone, Flupirtine, Fluproquazone, Flurbiprofen, Fosfosal, Gentisic Acid, Glafenine, Ibufenac, Imidazole Salicylate, Indomethacin, Indoprofen, Isofezolac, Isoladol, Isonixin, Ketoprofen, Ketorolac, p-Lactophenetide, Lefetamine, Loxoprofen, Lysine Acetylsalicylate, Magnesium Acetylsalicylate, Methotrimeprazine, Metofoline, Miroprofen, Morazone, Morpholine Salicylate, Naproxen, Nefopam, Nifenazone, 5' Nitro-2' propoxyacetanilide, Parsalmide, Perisoxal, Phenacetin, Phenazopyridine Hydrochloride, Phenocoll, Phenopyrazone, Phenyl Acetylsalicylate, Phenyl Salicylate, Phenyramidol, Pipebuzone, Piperylone, Prodilidine, Propacetamol, Propyphenazone, Proxazole, Quinine Salicylate, Ramifenazone, Rimazolium Metilsulfate, Salacetamide, Salicin, Salicylamide, Salicylamide O-Acetic Acid, Salicylsulfuric Acid, Salsalte, Salverine, Simetride, Sodium Salicylate, Sulfamipyrine, Suprofen, Talniflumate, Tenoxicam, Terofenamate, Tetradrine, Tinoridine, Tolfenamic Acid, Tolpronine, Tramadol, Viminol, Xenbucin and Zomepirac, as well as salts, esters, analogs, and derivatives thereof, and any combination thereof.

As used herein, a "pharmaceutically-acceptable salt" is understood to mean a compound formed by the interaction of an acid and a base, the hydrogen atoms of the acid being replaced by the positive ion of the base. Pharmaceutically-acceptable salts, within the scope of this disclosure, include both organic and inorganic types such as, for example, salts formed with ammonia, organic amines, alkali metal hydroxides, alkali metal carbonates, alkali metal bicarbonates, alkali metal hydrides, alkali metal alkoxides, alkaline earth metal hydroxides, alkaline earth metal carbonates, alkaline earth metal hydrides and alkaline earth metal alkoxides. Representative examples of bases that form such base salts include, but are not limited to, ammonia, primary amines such as n-propylamine, n-butylamine, aniline, cyclohexylamine, benzylamine, p-toluidine, ethanolamine and glucamine; secondary amines such as diethylamine, diethanolamine, N-methylglucamine, N-methylaniline, morpholine, pyrrolidine and piperidine; tertiary amines such as triethylamine, triethanolamine, N,N-dimethylaniline, N-ethylpiperidine and N-methylmorpholine; hydroxides such as sodium hydroxide; alkoxides such as sodium ethoxide and potassium methoxide; hydrides such as calcium hydride and sodium hydride; and carbonates such as potassium carbonate and sodium carbonate. Example of non-toxic acid addition salts include but are not limited to potassium, ammonium, hydrochloric, hydrobromic, hydroiodic, sulphate or bisulphate, nitrate, phosphate or hydrogen phosphate, acetate, benzoate, succinate, saccarate, fumarate, maleate, lactate, citrate, tartrate, gluconate, camsylate, methanesulphonate, ethanesulphonate, benzene-sulphonate, p-toluenesulphonate and pamoate salts. The compounds for use in the present disclosure can also provide pharmaceutically acceptable metal salts, in particular non-toxic alkali and alkaline earth metal salts, with bases. Examples include the sodium, potassium, aluminium, calcium, magnesium, zinc and diethanolamine salts. For a review on suitable pharmaceutical salts, see Berge et al, J. Pharm, Sci., 66, 1-19, 1977, incorporated herein by reference.

The particle size of the analgesic is important when formulating the powdered formulations of the present invention. For pulmonary or inhalation formulations, pharmaceutically acceptable respirable particles comprising a pharmaceutically active agent such as analgesic preferably have a size distribution which exhibits an aerodynamic diameter of less than 10 microns (less than 10 micrometers), more preferably less than 6 microns, and most preferably less than 5 microns. These may be referred to as micron-sized or micronized particles. More preferably, the micronized particles comprising the analgesic (referred to herein as drug particles or active particles) exhibit an equivalent aerodynamic diameter in the range of about 10.0, 9.5, 9.0, 8.5, 8.0, 7.5, 7.0, 6.5, 6.0, 5.5, 5.0, 4.5, 4.0, 3.5, 3.0, 2.5, 2.0, 1.5, 1.0, 0.9, 0.8, 0.7, 0.6, 0.5, 0.4, 0.3, 0.2, to 0.1 microns. In preferred embodiments, the mean diameter of the drug particles is about 0.5 to 6 microns. Throughout the present disclosure, the diameter of any of the particles referred to herein is the aerodynamic diameter of the particles. Prior to formulation, the micron-sized particles may be crystalline particles, amorphous particles, and combinations thereof. Preferably, the drug particles comprise at least about 98% analgesic, preferably at least about 99% analgesic, and most preferably at least about 99.5 % analgesic.

The drug particles in the desired particle size may be obtained by micronization according to methods well known in the art. Drug particles of the analgesic may also be prepared by jet milling, precipitation, recrystallization, precipitation from a solution in a first solvent system into a second solvent system wherein the first solvent system is completely soluble in the second solvent but the analgesic is not completely solvent in the second solvent system, rapid expansion from a solution of a supercritical fluid, precipitation into a liquefied gas, and other methods well known to those of skill in the art.

Micron-sized particles are generally thermodynamically unstable due to their high surface area to volume ratio, which may cause the particles to agglomerate. In an inhaler, agglomeration of micron-sized particles and adherence of particles to the walls of the inhaler are problems that result in the drug particles leaving the inhaler as large agglomerates or being unable to leave the inhaler and remaining adhered to the interior of the inhaler, both of which may result in unacceptably low respirable fraction of the emitted dose from the inhaler. But for powders to be reliably administered by an inhaler device, the carrier particles preferably should have a diameter greater than about 90 microns. Therefore, the particles administered by a powder inhalation device are preferably large while in the inhaler (drug particles carried on carrier particles), but small once introduced into the respiratory tract (drug particles separated from carrier particles).

To achieve this result, the powdered formulations of the present disclosure may comprise one or more pharmaceutically acceptable carriers, which can be taken into the respiratory tract with no significant adverse toxicological effects or swallowed and taken into the gastrointestinal tract with no significant adverse toxicological effects. Preferably the micron-sized particles of the analgesic adhere to the carrier while in the inhaler device, but are separated and dispersed from the surfaces of the carrier upon inhalation into the respiratory tract to give a fine particle size of the analgesic. Advantageously, substantially all (by weight) of the carrier particles have a diameter of between about 20 microns and about 1000 microns, more preferably between about 50 microns and about 1000 microns. In preferred embodiments, the diameter of substantially all (by weight) of the carrier particles is less than about 500, 450, 400, 350, 300, 250, 200, 190, 180, 170, 160, 150, 140, 130, 120, 110, 100, 90, 80, 70, 60, 50, 40, 30, or 20 microns. Preferably, the diameter of substantially all (by weight) of the carrier particles is from about 200 microns to 30 microns, more preferably from about 60 microns to about 180 microns, and most preferably from about 70 microns to about 130 microns in diameter. Substantially all (be weight) of the carrier particles means at least about 90% by weight of the carrier particles are of the desired range in diameter. It is well within the skill of one in the art to choose the appropriate size for the carriers found in the powdered formulations disclosed herein. The skilled artisan will understand that the size of the carrier particle must be suitably chosen for the specific drug being delivered to the patient in need of treatment.

The carrier may be composed of any pharmacologically inert material or combination of materials which is acceptable for inhalation. Preferably the carrier particles are water-soluble particles, which may be selected from one or more of sugars/carbohydrates (e.g., crystalline sugars, monosaccharides, disaccharides, sucrose, lactose, lactose monohydrate, glucose, glucose monohydrate, maltose, arabinose, saccharose, trehalose), oligo- and polysaccharides (e.g., dextrane), polyalcohols (e.g., sorbitol, mannitol, or xylitol), salts (e.g., salts of potassium, calcium, magnesium, sodium chloride or calcium carbonate) alpha hydroxyacids (e.g., citric acid, tartaric acid), polymers (e.g., polyvinyl alcohol, polyethylene glycol, hydrophilic celluloses, HPC, HPMC), peptides, amino acids, proteins and macromolecules, surfactants (e.g., sorbitan 20, sorbitan 80, and the like, in combination with a solid carrier), electrolytes (e.g., salts of potassium magnesium) and various non-electrolytes, or any combination thereof. The powdered formulations of the present disclosure may further comprise additive materials on the surfaces of the carrier particles to promote the release of the drug particles from the carrier particles upon release from the inhaler device or inhalation (see U.S. Patent No. 7,011,818, which is incorporated herein by reference). The skilled artisan will understand that the size of the carrier particle must be suitably chosen for the specific drug being delivered to the patient in need of treatment.

When a dose of a dry-powder pharmaceutical composition is inhaled, a percentage, such as a percentage in the range from about 1% to about 100%, more usually in the range from about 5% to about 90%, of the micron-sized drug particles in the dose are separated from the carrier. These micron-sized drug particles then deposit in the lung. If the forces causing the micron-sized drug particles to adhere on to other drug particles, carrier particles, or device surfaces increase or decrease from dose to dose, the therapeutically effective amount of the drug present in the powder composition will vary from dose to dose, resulting in clinical variation. Such variable adhesion also affects dose metering accuracy in reservoir devices. The methods of the present disclosure for preparing the powdered formulations disclosed herein provide for the preparation of a physically stable and homogeneous powdered formulation comprising particles of an analgesic that overcome these drawbacks.

U.S. Pat. No. 6,616,945 discloses a method for preparing a physically stable and homogeneous powdered preparation containing particles of an active agent, wherein the particles are suspended in a suspending solvent in which the particles are insoluble, and the suspending solvent is evaporated from the suspension. The active agents specifically identified in the '945 patent are salbutamol and budesonide. U.S. Pat. No. 6,881,422 relates to a methods of making a physically stable and homogenous powdered formulation comprising a tiotropium salt and a physiologically acceptable excipient by suspending both in a suspending solvent in which the particles are essentially insoluble, and removing the suspending solvent from the suspension step, for example by filtration, centrifugation, or evaporation. There are certain drawbacks in treating dry powders. For example, during vapor stabilization, the particles tend to fuse together to some extent, which must be controlled.

The powdered formulations of the present disclosure may be prepared by mixing the drug particles and carrier(s) in a dry homogenizer. Micron-size particles can exhibit high surface energy properties which permit the micron-sized particles to adhere strongly not only to each other but to the surfaces of other materials with which contact is made, such as with carrier particles, containers and device surfaces in which particle formulations reside, and the like. Consequently, in a formulation process involving a dry mixing of micron-sized active particles and carrier particles, care must be paid in the mixing process in order to obtain a homogenous distribution or blend of such drug particles on the surface of and adhesively proximal to or in contact with the carrier particles and/or to drug particles already adsorbed to the surface of the carrier particles. The drug particles may be added to the suspending solvent before the carrier particles, after the carrier particles, or at the same time as the carrier particles. A non-homogenous distribution of micron-sized particles and carrier particles or a non-homogeneous blend of such particles can produce unacceptable variations in the delivered dose (also referred to as total emitted dose) of the drug particles, especially when administered using reservoir devices, which additionally can show an inherent variation in accuracy of metering the dose.

The ratio in which the carrier particles and the drug particles are mixed will depend on the type of inhaler device used, the type of drug particles used, and the required dose. The percentage of drug particles with respect to carrier particles in the product blend can be from about 0.01% to about 50%, preferably from about 0.1% to about 30%, more preferably from about 0.2% to about 20%, more preferably from about 0.3% to about 10%, and most preferably from about 0.4% to about 10%. A dry mixture containing small amounts of micron-sized particles, such as less than 0.5% of drug particles with the remaining up to 99.5% as carrier particles, can be inhomogeneous with respect to drug particle distribution because of the presence of agglomerates of the drug, which can lead to lack of dose uniformity in a dry powder inhaler. Such agglomerates can be deagglomerated by application of ultrasound energy to a suspension of the drug particles and carrier particles in suspension of a suspending solvent, and the product blend will be substantially homogeneous and substantially free of agglomerated particles after evaporation of the suspending solvent.

In a preferred embodiment, the present disclosure provides a method for preparing a physically and chemically stable and homogenous powdered formulation suitable for pulmonary or inhalation comprising a blend of (a) dry micron-sized particles comprising fentanyl, preferably in the form of a fentanyl salt, and most preferably in the form of fentanyl citrate, and (b) a pharmaceutically acceptable carrier in particulate form suitable for pulmonary or inhalation use, which blend is made suitable for pulmonary inhalation and provides for pulmonary administration of the fentanyl particulates. The molecular weight of fentanyl as a free base is 336.5 g/mole. The molecular weight of fentanyl citrate salt is 528.6 g/mole. Thus, 157.09 µg of fentanyl citrate in a powdered formulation is equivalent to or provides the equivalent of 100 µg of fentanyl as the free base in the formulation.

A preferred method for preparing the powdered formulations of the present disclosure is characterized in that the particles of an analgesic are suspended in a suspending solvent in which the particles are essentially insoluble, sparingly soluble, slightly soluble, or partially soluble, and the suspending solvent is removed from the suspension, which generates a physically and chemically stable, homogeneous powder preparation. As used herein, "essentially insoluble", "sparingly soluble", "slightly soluble", and "partially soluble" all fall within the range of at least 30 parts solvent to dissolve one part solute. The suspending solvent may be removed by means well known to those of skill in the art, including but not limited to, evaporation, filtration, spray drying, and/or centrifugation. When the suspending solvent is removed from the suspension through evaporation, this is preferably accomplished through an evaporator, a rotary evaporator, distillation, vacuum distillation, or inert gas purging. After drying, the powder may optionally be sieved. Although not wishing to be bound to any theory, a benefit of the suspending solvent may be that it prevents agglomeration of the particles during recrystallization, thereby providing a stable and homogenous formulation. The suspending solvent should be chemically inert in respect of the formulation components, fairly volatile, and the components must be insoluble, sparingly soluble, slightly soluble, or partially soluble in the suspending solvent.

The suspending solvent may be selected from the group consisting of alkanes, alcohols, ketones, hydrocarbons, halogenated hydrocarbons (e.g., CFC 11 and HFC 227), or any mixtures thereof. More particularly, the suspending solvent may be selected from the group consisting of acetane, acetone, benzophenone, butanol, butan-2-one, carbon tetrachloride, chloroform, cyclohexane, cyclohexanone, 1-chloropropane, 2-chloropropane, 1-chlorobutane, 2-chlorobutane, 1-chloro-2-methylpropane, 1-chloropentane, 1,1-dichloroethane, 1,2-dichloroethane, dichloromethane, dodecanol, ethanol, ethylchloride, hexane, hexanol, heptane, isopropanol, methanol, methyl ethyl ketone, neopentanol, phenol, 2,6-di-i-propylphenol, n-propanol, pentane, t-butanol, 4-t-octylphenol, 5-norbornene-2-methanol, and mixtures thereof. Additionally, the suspending solvent may be selected from the group consisting of:
(a) C₄ to C₈ alkanes which can be linear, branched or cyclic;
(b) C₁ to C₈ halogenated alkanes of the formula CₙH₍₂ₙ₋ₓ₊₂₎Xₓ, wherein x can be from 1 to 2n+2, which halogenated alkanes can be linear, branched or cyclic, and wherein the halogen, X, is preferably selected from the group consisting of bromine, chlorine, fluorine, and combinations thereof;
(c) alcohols of the formula ROH, which can be linear, branched or cyclic;
(d) ketones of the formula R₁(CO)R₂, which can be linear, branched or cyclic; and.
(e) mixtures thereof.

In certain embodiments of the present disclosure, the suspending solvent is selected from the above groups (a) and (e), and is capable of forming azeotropes in the presence of water. An azeotrope is a special homogenous mixture of two or more compounds (molecules), wherein the ratio of the compounds is exactly the same in both the vapour form of the mixture as in the liquid phase (e.g., hexane has a boiling point of 69.0°C and water has a boiling point of 100°C at one atmosphere, and can form an azeotrope which boils at 61.6°C and contains 94.4% hexane and 5.6% water; butane has a boiling point of -0.5°C; pentane has a boiling point of 36.1°C and can form an azeotrope with water which boils at 34.6°C and contains 98.6% pentane and 1.4% water, and which can separate into an upper layer which contains 99.95% pentane and 0.05% water and a lower layer which contains 0.04% pentane and 99.96% water; heptane has a boiling point of 98.4°C and forms an azeotrope with water which boils at 79.2°C and contains 87.1% heptane and 12.9% water, and which can separate into an upper layer which contains 99.98% heptane and 0.02% water, and a lower layer which contains 0.01 % heptane and 99.99% water).

In other embodiments, the suspending solvent may comprise a combination of two or more solvents, for example a first solvent and a second solvent. For example, the suspending solvent may comprise a combination of a first solvent with a dielectric constant of less than about 10, and a second semi-polar solvent with a dielectric constant of equal to or greater than about 10 and less than about 50. In other embodiments, the dielectric constant of the first solvent is less than about 9.5, 9.0, 8.5, 8.0, 7.5, 7.0, 6.5, 6.0, 5.5, 5.0, 4.5, 4.0, 3.5, 3.0, 2.5, 2.0, 1.5, 1.0 or 0.5. In still other embodiments, the dielectric content of the second semi-polar solvent is greater than about 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40, and less than about 49, 48, 47, 46, 45, 44, 43, 42, 41, 40, 39, 38, 37, 36, 35, 34, 33, 32, 31, 20, 29, 28, 27, 26, 25, 24, 23, 22, 21, or 20, or any ranges in that fall between the above values. It is well within the skill of one in the art to appropriately combine a non-polar first solvent and a semi-polar second solvent to formulate the powdered formulations disclosed herein. A particularly preferred suspending solvent is a mixture of hexane and alcohols, more preferably a mixture of n-hexane and 2-butanol.

Agitation of the suspending solvent and the suspension of particles in the suspending solvent is preferably maintained by mechanical stirring, ultrasonication, or a combination thereof. Agitation can provide a means to thoroughly mix and distribute all components of the suspension. Agitation can also deagglomerate clusters of small particles in the suspending solvent to provide a effectively smaller and narrower particle size distribution of the micron-sized drug particles in the suspension. A preferred method of agitation comprises application of ultrasound energy to the suspended particles. It is well within the skill of one in the art to appropriately choose the agitation parameters to formulate the powdered formulations appropriate for inhalation disclosed herein. The manufacturing process for the powdered formulations disclosure herein may be done in a totally closed system in the absence of water. The process is very safe in respect to any contamination and can be used in large-scale production. The mixing vessel can be equipped with mechanical stirrer, ultrasonic transmitter, heaters and filters, and vacuum evaporation so that the formulation is ready to use after it is processed.

When micronized fentanyl citrate and a lactose carrier were suspended into the suspending solvent n-hexane, a non-polar suspending solvent (dielectric constant of 1.89), the fentanyl particles did not easily disperse into the liquid, and were poorly wetted. When the suspending solvent was subsequently evaporated to obtain an inhalation powder, the powder possessed a low bulk density. Although this final formulation had a relatively fine particle fraction, it also had poor flowability and poor homogeneity. The properties of the resulting powder formulation were improved by adding a second semi-polar suspending solvent, 2-butanol (dielectric constant of 15.8), to the first non-polar suspending solvent, thereby sufficiently increasing the polarity of the suspending solvent. The particles of fentanyl citrate and lactose are sufficiently soluble in this suspending solvent such that the resulting powder formulation has good flowability and the drug is homogeneously distributed with the carrier. The preferred suspending solvent comprised a mixture of 90:10 n-hexane and 2-butanol, respectively. The powdered formulation produced using this suspending agent was well flowing, physically and chemically stable and homogenous, with increased bulk density. The physical properties of the micron-sized fentanyl particles, and the physical interactions of the fentanyl particles with the carrier particles, are improved in such a way that results in reproducible production of the powdered formulation. In addition, the resulting powdered formulation has a lower fine particle fraction. In addition, the pharmacokinetic profile of the powdered formulation is improved for the delivery of this potent analgesic to treat severe pain, i.e., pulmonary or inhalation administration of the powdered formulation result in essentially immediate absorption combined with a prolonged stable systemic concentration.

More specifically, the method is characterized in that the micron-sized drug particles comprising fentanyl and the carrier particles are together admixed and suspended with agitation for an agitation time in a suspending solvent in which the fentanyl-containing micron-sized particles and the carrier particles and the admixture of such particles are each essentially insoluble or sparingly soluble, to form an agitated suspension. From the thus obtained agitated suspension, the suspending solvent is subsequently removed, for example by evaporation under controlled temperature and pressure conditions, to provide a homogeneous product blend of drug particles comprising fentanyl and carrier particles, which when administered by pulmonary inhalation may provide fentanyl in a form that is highly bioavailable when compared to oral mucosal delivery of fentanyl citrate, but not as highly bioavailable as an intravenous injection which may be associated with undesirably high peak concentration and undesirably short duration of action.

Preferably, the product blend, i.e., powdered formulation, maintains excellent particle composition homogeneity during subsequent storage and manipulation, including during transportation, storage, packaging (e.g., packaging into an inhalation device), and shelf storage, and during the period of repeated use by a patient of the device for inhalation administration of the product blend. The powdered formulation can be administered to the patient by means of a metered dose inhalation device to provide reproducible doses, and is compositionally and physically stable with respect to exposure to conditions of increased temperature and humidity when contained in an inhalation device. The metered dose inhalation device can be breath actuated. The percentage of drug particles comprising fentanyl with respect to carrier particles in the product blend can be from about 0.01 % to about 50%, preferably from about 0.1 % to about 30%, more preferably from about 0.2% to 20%, more preferably from about 0.3% to about 10%, and most preferably from about 0.4% to about 10%.

The formulation of the present disclosure comprising one or more analgesics as the active agent is preferably in the form of a powder formulated for pulmonary or inhalation delivery. Such a powdered formulation is preferably delivered or administered by means of an inhaler device. Inhalers are well known devices for administering pharmaceutical products to the respiratory tract by inhalation. Among inhalation devices useful for delivery of powdered formulations are dry powder inhalers. These devices preferably comprise a powder reservoir and means for metering a dose from the reservoir for each delivery to the patient, as well as devices provided with a pre-metered powder dose, for example, a dose contained in a capsule, in a blister, and the like. The performance of any suitable powder inhaler should fulfill the requirements of Pharmacopeias for delivered dose uniformity (e.g., plus or minus 25% of the mean dose). Generally, the mass of respirable particles per dose administered by an inhaler must be consistent among actuations and administrations. In addition, the stability of the powdered formulation with respect to keeping as a function of, for example, time, temperature, and relative humidity, should reside within limits which provide reproducible doses of active agent.

A preferred inhalation device for administering the powdered formulations disclosed herein can be selected from the group consisting of single-dose disposable inhalers, single-dose multi-use inhalers, multi-use pre-metered inhalers, multi-use reservoir based inhalers, and other dry powder inhalers. Preferable inhalation devices are breath actuated. Dry powder dispersion devices have been described in a number of patent documents, including but not limited to EP467172; WO91/02558; WO93/09832; U.S. Pat. Nos. 3,921,637, 4,627,432, 4,811,731, 5,035,237, 5,048,514, 4,446,862, 5,048,514, and 4,446,862. In a most preferred embodiment, the inhalation device is a TAIFUN^{®} inhalation device, which is described in U.S. Pat. Nos. 6,132,394, and 5,476,093, the entire contents of which are herein incorporated by reference. The TAIFUN® technology platform has been previously incorporated into a salbutamol powder inhaler, which received marketing authorizations in ten European countries.

The device, such as TAIFUN® device or other metered dose inhaler device, can be charged with sufficient powdered formulation comprising an analgesic, such as fentanyl or fentanyl citrate, to provide a sufficient amount of the powdered formulation to permit repeated administration of doses of the analgesic, for example, repeated doses of between 50 µg to 800 µg of analgesic, including, for example, sufficient blend for a repeated 50 µg dose of analgesic, a repeated 100 µg dose of analgesic, a repeated 150 µg dose of analgesic, a repeated 200 µg dose of analgesic, a repeated 250 µg dose of analgesic, a repeated 300 µg dose of analgesic, a repeated 350 µg dose of analgesic, a repeated 400 µg dose of analgesic, a repeated 450 µg dose of analgesic, a repeated 500 µg dose of analgesic, a repeated 550 µg dose of analgesic, a repeated 600 µg dose of analgesic, a repeated 650 µg dose of analgesic, a repeated 700 µg dose of analgesic, a repeated 750 µg dose of analgesic, or a repeated 800 µg dose of analgesic.

Titration of breakthrough pain, especially in opioid-tolerant patients, may be accomplished by pulmonary inhalation of a powdered formulation comprising analgesic drug particles, such as one or two administrations of 100 µg or 200 µg doses, preferably within 15 minutes of each other.

If a single-dose dry powder inhalation device such as a disposable single-use device is provided to a patient, it may contain one dose of from about 50 µg to about 800 µg, preferably from about 100 µg to about 400 µg, and most preferably from about 100 µg to about 200 µg. Titration of breakthrough pain, especially in opioid tolerant patients, can then be accomplished by pulmonary inhalation of powdered formulation disclosed herein using one of more separate single-use devices in succession, preferably within 15 minutes of each other.

If a multi-dose dry powder inhalation device such as a TAIFUN® inhaler is used, the device can be manufactured to reproducibly meter repeated doses such as repeated doses of about 50 µg of fentanyl, or repeated doses of about 100 µg of fentanyl, or repeated doses of about 200 µg of fentanyl, or repeated doses of about 400 µg. A patient, especially an opioid tolerant patient, can be titrated for treatment of breakthrough pain by repeated inhalation of 50 µg to 400 µg doses of a powdered formulation of the present disclosure comprising fentanyl, or more preferably by repeated inhalation of 100 µg to 200 µg doses of such formulation.

It has been discovered that at least about 85%, 90%, 91%, 92%, 93% 94%, 95%, and up to about 96% of all patients treated, especially of opioid tolerant patients, suffering from pain can be titrated to obtain satisfactory pain relief by administration of one dose of 100 µg to 400 µg, or two doses of 100 µg to 200 µg of the powdered formulation of the present disclosure comprising fentanyl (with a 4-fold titration range) by pulmonary administration. It has also been discovered that at least about 25%, 30%, 31%, 32%, 33% 34%, 35%, 36%, 37%, 38%, and up to 39% of all patients, especially opioid tolerant patients, suffering from breakthrough pain can be titrated to obtain satisfactory pain relief by administration of one dose of 100 µg of the powdered formulation comprising fentanyl of the present disclosure by pulmonary administration. It has further been discovered that at least about 65%, 68%, 70%, 71%, 72%, 73% and up to 74% of all patients, especially opioid tolerant patients, suffering from breakthrough pain can be titrated to obtain satisfactory pain relief by administration of one dose of 200 µg of the powdered formulation comprising fentanyl of the present disclosure by pulmonary administration. Optionally, multiple doses such as two 100 µg doses may be administered.

Thus, one embodiment of the present disclosure provides a method to titrate breakthrough pain, such as breakthrough pain caused by cancer or by progression of a disease such as cancer, in at least about 25%, 30%, 31%, 32%, 33% 34%, 35%, 36%, 37%, 38%, and up to about 39% of all patients, especially opioid tolerant patients, by pulmonary inhalation administration of 100 µg (such as by administration of one dose of 100 µg or two doses of 50 µg) of the powdered formulation comprising fentanyl of the present disclosure, preferably by administration as Fentanyl-TAIFUN®.

In another aspect, this invention provides a method to titrate breakthrough pain, such as breakthrough pain caused by cancer or by progression of a disease such as cancer, in from about 39% to 74% of all patients suffering from breakthrough pain, especially in opioid tolerant patients, by pulmonary inhalation administration of 200 µg (such as by administration of one dose of 200 µg, or by administration of two doses of 100 µg, and the like to a total of 200 µg) of fentanyl-containing micron-sized particles prepared according to this invention, preferably by administration of such fentanyl-containing micron-sized particles in the form of Fentanyl-TAIFUN®.

In another aspect, this invention provides a method to titrate breakthrough pain, such as breakthrough pain caused by cancer or by progression of a disease such as cancer, in from about 39% to 74% of all patients suffering from breakthrough pain, especially in opioid tolerant patients, by pulmonary inhalation administration of two doses of 100 µg of fentanyl-containing micron-sized particles prepared according to this invention, preferably by administration of such fentanyl-containing micron-sized particles in the form of Fentanyl-TAIFUN®.

In another aspect, this invention provides a method to titrate breakthrough pain, such as breakthrough pain caused by cancer or by progression of a disease such as cancer, in from about 74% to 96% of all patients suffering from breakthrough pain, especially in opioid tolerant patients, by pulmonary inhalation administration of 400 µg (such as by administration of two doses of 200 µg or four doses of 100 µg) of fentanyl-containing micron-sized particles prepared according to this invention, preferably by administration of such fentanyl-containing micron-sized particles in the form of Fentanyl-TAIFUN®.

In another aspect, this invention provides a method to titrate breakthrough pain, such as breakthrough pain caused by cancer or by progression of a disease such as cancer, in from 74% to 96% of all patients suffering from breakthrough pain, especially in opioid tolerant patients, by pulmonary inhalation administration of four doses of 100 µg of fentanyl-containing micron-sized particles or two doses of 200 µg of fentanyl-containing micron-sized particles prepared according to this invention, preferably by administration of such fentanyl-containing micron-sized particles in the form of Fentanyl-TAIFUN®.

In another aspect, this invention provides a method to titrate breakthrough pain, such as breakthrough pain caused by cancer or by progression of a disease such as cancer, in from 96% to 100% of all patients suffering from breakthrough pain, especially in opioid tolerant patients, by pulmonary inhalation administration of greater than 400 µg (such as up to 600 µg, up to 800 micrograms, up to 1200 µg, or up to 1600 µg for opioid resistant patients) of fentanyl-containing micron-sized particles prepared according to this invention, preferably by administration of such fentanyl-containing micron-sized particles in the form of Fentanyl-TAIFUN®.

The following examples are included to demonstrate preferred embodiments of the present disclosure without any limitation thereto.

### Example 1

Preparation of a fentanyl citrate formulation which provides 100 µg doses of fentanyl.

Micronized fentanyl citrate particles (20.3 g) with a mean particle size of about 2 to 4 microns were suspended in a 10.2 w/w% solution of 2-butanol (87.5 g) in n-hexane (770 g) as a suspending solvent, with stirring at 18-24°C and at ambient pressure to form a mobile slurry. Ultrasonic energy was applied using a custom made ultrasound device for 5 (0-10) minutes. Lactose monohydrate (580.3 g) was added to the mechanically stirred suspension of fentanyl citrate particles at 18-24°C, and stirring was continued for 5 minutes at 18-24°C. The suspending solvent was then removed by filtration at 20-100 mbar pressure drop, and subsequent evaporation in an evaporator at reduced pressure (900-1 mbar) at 40-60°C for 3 hours. The dry powder was passed through a sieved resulting in a free-flowing powder. The free-flowing powder exhibited RSD values of 0.39 to 1.41%.

### Example 2

Preparation of a fentanyl citrate formulation which provides 200 µg doses of fentanyl.

Micronized fentanyl citrate particles (44.1 g) with a mean particle size of about 2 to 4 microns were suspended in a 10.2 w/w% solution of 2-butanol (87.5g) in n-hexane (770 g), with stirring at 18-24°C and ambient pressure, to form a mobile slurry. Ultrasonic energy, an external energy means, was applied using a custom made ultrasound device for 5 (0-10) minutes. Lactose monohydrate (557.3 g) was added to the mechanically stirred suspension of fentanyl citrate particles at 18-24°C, and stirring was continued for 5 minutes at 18-24°C. The suspending solvent was then removed by filtration at 20-100 mbar pressure drop, and subsequent evaporation in an evaporator at reduced pressure (900-1 mbar) at 40-60°C for 3 hours. The dry powder was passed through a sieved resulting in a free-flowing powder. The free-flowing powder exhibited RSD values of 0.83 to 2.59%.

Prior to administration, the free-flowing powder was loaded into a TAIFUN® inhaler, a device useful for pulmonary inhalation delivery of the fentanyl powder composition. The amount of free-flowing powder containing fentanyl citrate was sufficient to deliver up to thirty 100 µg doses of fentanyl by inhalation.

The respirable fraction in a delivered dose was approximately 20-40% of the delivered dose, with a delivered dose uniformity of<10% RSD. The formulation exhibits stability at room temperature for over 2 years. The formulation exhibits stability at 40°C, 75% relative humidity (RH), for over 4 months. No change in the particle size distribution or respirable fraction in the delivered dose was seen when tested after 2 and 4 weeks. These characteristics of the formulation are repeatable from batch to batch.

### Example 3

Preparation of a fentanyl citrate formulation which provides 50 µg doses of fentanyl.

Micronized fentanyl citrate particles (9.4 g) with a mean particle size of about 2 to 4 microns were suspended in a 3.0 w/w% solution of 2-butanol (23.8 g) in n-hexane (770 g) as a suspending solvent, with stirring at 18-24°C and at ambient pressure to form a mobile slurry. Ultrasonic energy was applied using a custom made ultrasound device for 5 (0-10) minutes. Lactose monohydrate (580.3 g) was added to the mechanically stirred suspension of fentanyl citrate particles at 18-24°C, and stirring was continued for 5 minutes at 18-24°C. The suspending solvent was then removed by filtration at 20-100 mbar pressure drop, and subsequent evaporation in an evaporator at reduced pressure (900-1 mbar) at 40-60°C for 3 hours. The dry powder was passed through a sieved resulting in a free-flowing powder. The free-flowing powder exhibited RSD values of 0.58 to 1.69%.

### Example 4

Animal studies in rats and dogs using an inhaled formulation of fentanyl citrate (Fentanyl TAIFUN®).

The dry powder fentanyl formulations as disclosed herein were administered by inhalation via the pulmonary route using the inhalation device TAIFUN®, which is described in U.S. Patent Nos. 6,132,394 and 5,476,093, each of which is incorporated herein by reference. The administration of the fentanyl formulations disclosed herein using the TAIFUN® device is referred to hereinafter as Fentanyl TAIFUN®. Repeated inhalative administration of Fentanyl TAIFUN® up to 28 days and up to maximum tolerated dose levels in rats and dogs (300 µg/kg/day in the rat; 40 µg/kg/day in the dog) was tolerated without target organ toxicity. Some pharmacological clinical signs (like salivation, decreased or increased activity, increased muscle tone (rats), abnormal breathing pattern) were observed dose-dependently at all doses. Minimal adaptive histopathological changes were observed in the respiratory epithelium (hypertrophy/hyperplasia of epithelial and goblet cells) or in the lungs of rats (focal inflammatory reactions) at fentanyl plasma levels which exceeded several times those expected in human opioid-naive patients (ca. 3- to 7-fold in dogs and approximately 10-to 100-fold in rats). Other toxicological studies raised no further concerns.

### Example 5

Phase I clinical trial data obtained in 24 male volunteers using an inhaled formulation of fentanyl citrate (Fentanyl TAIFUN®).

A clinical Phase I study was performed in twenty-four healthy male volunteers to examine the pharmacokinetic dose linearity and safety of a dry powdered formulation of fentanyl citrate produced as set forth in Example 3. The dry powder fentanyl formulations were administered by inhalation via the pulmonary route using the inhalation device TAIFUN® (Fentanyl TAIFUN®). The study demonstrated that fentanyl was rapidly absorbed after a single pulmonary application; for pulmonary application of 50 µg fentanyl the maximum fentanyl concentration was recorded at the first measurement point 5 minutes after administration. Plasma concentrations increased proportionally with increasing dose, and the absolute bioavailability of fentanyl from Fentanyl TAIFUN® was high. In general, Fentanyl TAIFUN® was well tolerated. Also, previous inhalation studies in volunteers with other devices suggest that the adverse effects are of the same quality and intensity that occur after for example, intravenous dosing. Respiratory depression can occur at all therapeutic plasma concentrations, as is the case with all the other administration routes, and should be carefully monitored.

### Example 6

Phase I clinical trial data obtained in 30 volunteers using an inhaled formulation of fentanyl citrate (Fentanyl TAIFUN®).

A clinical Phase I open-label, randomized, 5-period, single-escalating dose study was performed in thirty healthy volunteers to examine the pharmacokinetic dose linearity and safety of a dry powdered formulation of fentanyl citrate produced as set forth in Examples 1 and 2. The dry powder fentanyl formulations were administered by inhalation via the pulmonary route using the inhalation device TAIFUN® (Fentanyl TAIFUN®). The study compared the bioavailability of fentanyl after pulmonary application of 100 microgram (µg), 200 µg, 400 µg, and 800 µg doses of fentanyl from the TAIFUN® device, relative to a single oromucosal dose of Actiq® 200 µg fentanyl (a 200µg compressed lozenge with integral oromucosal applicator). Actiq® 200 µg, the study reference medication, is a licensed medication indicated for the management of breakthrough pain in patients receiving maintenance opioid therapy for chronic cancer pain. The study was performed in compliance with the ICH Harmonised Tripartite Guideline for Good Clinical Practice (GCP).

The thirty healthy volunteers were non-smokers between the ages of 18 and 50 years, with a Body Mass Index (BMI) of 20 to 28 kg/m², weighing between 60-90 kg. The volunteers exhibited no clinically significant laboratory test results or abnormalities in a 12-lead electrocardiogram (ECG). To be eligible for the study, the volunteers provided written informed consent to participate in the study, satisfied a medical examiner about their fitness to participate in the study, and were required to be available to complete the study. A sample size of thirty subjects (for a 5 phase study (4 test dose levels)) is sufficient to ensure a 2-sided 95.0% confidence interval for the regression slope of log (AUCT) versus log (dose) will extend approximately 0.1 from a predicted slope of 1.10. This is assuming a standard deviation of 0.77 for the predictor variable (dose) and that the standard deviation for the residuals is 0.645.

Fentanyl TAIFUN® is a novel dry powder inhaler, with the fentanyl powdered formulation specially manufactured for the inhaler. For use in this study, each inhaler was formatted to deliver thirty doses of fentanyl by the pulmonary route. It is contemplated that the Fentanyl TAIFUN® inhaler may be produced to contain any therapeutic dose strength of fentanyl, including but not limited to doses of 50 µg, 100 µg, 150 µg, 200 µg, 250 µg, 300 µg, 350 µg, 400 µg, 450 µg, 500 µg, 550 µg, 600 µg, 650 µg, 700 µg, 750 µg, 800 µg, or higher doses of fentanyl.

The doses used for testing in this study were 100µg, 200µg, 400µg, and 800µg of fentanyl. These doses were delivered from the Fentanyl TAIFUN® inhaler in the following manner: a 100 µg dose of fentanyl was delivered as one inhalation from a 100µg/dose Fentanyl TAIFUN® inhaler; a 200 µg dose of fentanyl was delivered as one inhalation from a 200µg/dose Fentanyl TAIFUN® inhaler; a 400 µg dose of fentanyl was delivered as two inhalations from a 200µg/dose Fentanyl TAIFUN® inhaler; and a 800 µg dose of fentanyl was delivered as four inhalations from a 200µg/dose Fentanyl TAIFUN® inhaler.

Each dose of study medication in the Phase I study was administered following pre-medication with 50 mg of naltrexone (Nalorex^{®}). Naltrexone is an opioid antagonist treatment medication, which acts as a pure, potent mu antagonist, with minimal side effects. It is neither reinforcing nor addicting and has no potential for abuse or diversion for unprescribed use.

Subjects were allocated to one of five treatment sequence groups according to a randomization code. The five treatment sequences are described below in Table 1. During the study, there was a minimum of seven days between doses. The term "Reference" as used in this study refers to the study reference medication, Actiq® 200 µg.

**Table 1**

| | **Sequence Group 1** | **Sequence Group 2** | **Sequence Group 3** | **Sequence Group 4** | **Sequence Group 5** |
|---|---|---|---|---|---|
| **Dosing Occasion 1** | 100 µg | 100 µg | 100 µg | 100 µg | Reference |
| **Dosing Occasion 2** | 200 µg | 200 µg | 200 µg | Reference | 100 µg |
| **Dosing Occasion 3** | 400 µg | 400 µg | Reference | 200 µg | 200 µg |
| **Dosing Occasion 4** | 800 µg | Reference | 400 µg | 400 µg | 400 µg |
| **Dosing Occasion 5** | Reference | 800 µg | 800 µg | 800 µg | 800 µg |

Doses were administered to each subject under medical supervision in the morning at approximately 9 am on Study Day 1. Subjects were required to fast for at least 8 hours overnight prior to each morning dose on Study Day 1. The fast was broken 4 hours after dosing with a light lunch. Subjects were provided a meal 8 hours after dosing, and a light snack 12 hours after dosing.

On Study Day 2, 24 hours after dosing, the subject was provided with breakfast following the completion of a 24-hour post-dose assessment. Subjects received an initial dose of 100µg Fentanyl TAIFUN® or 200 µg Reference medication. Subsequent doses were 200 µg, 400 µg and 800 µg Fentanyl TAIFUN® or 200 µg Reference medication, as shown in Table 1. One hour prior to dosing, subjects were pre-medicated with 50 mg naltrexone (Nalorex®). Dose escalation continued until the maximum proposed dose of 800 µg Fentanyl TAIFUN® was administered.

For administration of the Reference medication, the subject placed a Actiq® 200 µg unit in their mouth between the cheek and lower gum, occasionally moving the drug unit from one side to the other using the handle. The Actiq® unit was sucked, not chewed. A unit dose of Actiq®, if chewed and swallowed, might result in lower peak concentrations and lower bioavailability than when consumed as directed. The subjects were instructed to consume the Actiq® unit over a 15-minute period. Longer or shorter consumption times could produce less efficacy than reported in Actiq® clinical trials. In some instances, certain subjects consumed the Actiq® unit in times longer or shorter than the instructed time of 15 minutes. All subjects completely consumed the Actiq® unit.

For the test product Fentanyl TAIFUN®, the pharmacokinetic parameters evaluated statistically include Cmax, Tmax, AUC, AUCT, AUCI, and t_{1/2} The Cmax, AUCT and AUCI were log-transformed (base e) prior to analysis, and were tested for dose-proportionality of the test product by using a mixed effects model, with subject as the random effect of the log-transformed parameter versus the log transformed (base e) dose. The AUC0-t, AUC0-∞ and Cmax were assessed for dose-proportionality using a mixed effects model, with subject as the random effect of the log-transformed parameter versus the log-transformed (base e) dose. For each parameter, a point estimate and 95% confidence interval were calculated for the slope of the regression line.

For the test product Fentanyl TAIFUN®, t_{1/2} was tested for dose independence using a mixed effects model, with subject as the random effect of the parameter versus dose. A point estimate and 95% confidence interval were calculated for the slope of the regression line. Tmax was analyzed non-parametrically using the Wilcoxon matched pairs test. Following logarithmic transformation, Cmax, AUCT, AUC0-t, AUC0-∞, and AUCI values were subjected to an analysis of variance (ANOVA) for all treatments. The terms included in the model were sequence, subjects nested within sequence, and administration. The 95% confidence intervals were constructed for each of the test doses against the Reference dose. The point and interval estimates were then back-transformed to give estimates of the ratio of the geometric least squares means and the corresponding 95% confidence intervals.

Of forty-four subjects who consented to participating in the study, and were screened and randomized for participation, thirty were dosed on at least one occasion with either the test formulation of Fentanyl TAIFUN® or the Reference formulation. The subjects were allocated to treatment groups according to a randomization code produced using the PROC PLAN procedure of SAS Version 9.1.3. All thirty subjects who were randomized were assessed for adverse events and laboratory test abnormalities. Two subjects were excluded from the pharmacokinetic population because they only received two doses of the study medication. During the study, a total of 142 dosing occasions occurred within a forty-six day period. The disposition of the subjects is shown below in Table 2.

**Table 2**

| | Number of Subjects | | | | |
|---|---|---|---|---|---|
| | Reference | 100 µg | 200 µg | 400 µg | 800 µg |
| Number Screened (n = 44) | | | | | |
| Safety Population | 29 | 30 | 29 | 28 | 26 |
| PK Population | 28 | 28 | 28 | 28 | 26 |
| Withdrawn due to Adverse Event | 0 | 1 | 1 | 2 | 0 |
| Completed | 29 | 29 | 28 | 26 | 26 |

Two subjects were withdrawn from the study following the administration of two doses of study medication due to adverse events. Two further subjects were withdrawn due to adverse events following the administration of four doses of study medication due to adverse events.

Blood samples (6 mL) from the subjects for determination of fentanyl plasma levels were collected in Lithium Heparin sampling tubes at the following times: pre-dosing, 1, 3, 5, 10, 20, 30, and 45 minutes after the end of dosing, and 1, 2, 4, 8, 12, 24, 48, and 72 hours after the end of dosing. Samples were taken via a peripherally placed intravenous cannula, which was kept patent with 1 mL saline flushes. The cannula remained *in situ.* The first 1 mL of blood collected from the subject at each time point was discarded in order to remove the saline. The maximum total amount of blood withdrawn for pharmacokinetic analysis from each subject was 5 mL x 16 samples x 5 dosing periods, for a total of 480 mL, over the 5 dosing periods, plus blood required for pre-study screening, post-study follow up, and serum pregnancy test for female volunteers on each dosing phase (approximately 21 mL at pre-study screening and at post-study follow up, and approximately 4.5 mL at each dosing phase for female volunteers), for approximately 545 mL in total.

Immediately upon sampling the sample was identified with a bar coded label bearing details of study number, subject number, sampling time point, sample type and a unique 9-digit identification number. The sample was separated within 30 minutes of collection by centrifugation at 1500 x g at 4°C for 10 minutes. Two equal aliquots of plasma were transferred to polypropylene tubes labeled identically to the original blood sample and stored at approximately -20°C pending analysis. The times at which samples were taken, received into the separating room, and placed in the freezer were recorded in study documentation.

Following solvent-extraction from the study plasma samples, along with a deuterated internal standard, fentanyl was determined by capillary gas chromatography - positive ion chemical ionization mass spectrometry over the concentration range of 10 to 2000 pg/mL, using a validated method. The following pharmacokinetic parameters were derived from the plasma fentanyl concentration by non-compartmental analysis: Cmax, Tmax, AUCT, AUCI, AUC 0-20, AUC 0-30, AUC 20-I, AUC 30-I, and t½. The pharmacokinetic assessment variables included:
Cmax = Maximum plasma concentration [pg/mL];
Tmax = Time [hours] at which Cmax occurs;
AUC = Area under the plasma concentration versus time curve;
AUCT = AUC [ng/mL*hr] during a dosing interval t in steady state (from time = 0 hours to time = T hours);
AUCI = AUC [ng/mL*hr] time = 0 hours extrapolated to infinity (I); and
t_{1/2} = Elimination half-life [hours].
The derivation of these variables from the raw plasma concentrations was determined using WinNonlin Professional v2.1 computer software.

Pharmacokinetic parameters and plasma concentrations of fentanyl were summarized. The summary statistics obtained include the geometric mean, geometric CV (%), arithmetic mean, arithmetic SD, CV (%), median, min, max and N. The geometric mean was not presented for Tmax. Mean and individual plasma concentration-time curves of fentanyl were derived on both linear and semi-logarithmic scales, and derived pharmacokinetic parameters were obtained graphically, as appropriate. All pharmacokinetic parameters (e.g., AUC0-t, AUC0-∞ and Cmax), except t½ and Tmax, were log-transformed (base e) prior to statistical analysis. An additional test for a period effect was carried out using a one-way ANOVA for each analysis/parameter. The validity of each analysis was assessed by the inspection of residuals from the model.

The safety population consisted of all subjects who received at least one dose of study medication. The pharmacokinetic population consisted of those subjects who were dosed with at least three doses of study medication provided they had received the Reference treatment and had sufficient blood samples to obtain a plasma concentration by time profile.

The plasma fentanyl concentrations following the administration of the Reference medication (Actiq®) showed a slow rise in the plasma concentration to reach a mean peak (Cmax) of 401.84 pg/mL at a median time (Tmax) of 1 hour. The plasma fentanyl concentrations following the administration of inhaled fentanyl as Fentanyl TAIFUN® at 100µg, 200µg, 400µg and 800µg showed a rapid rise in the plasma concentration to reach an average peak of 610.97 pg/mL, 1146.99 pg/mL, 1870.42 pg/mL, and 3470.85 pg/mL, respectively, with a variation between 1 minute (0.017h) and 3 minutes (0.050h) in median time (Tmax) post dosing. Fentanyl from the Fentanyl TAIFUN® formulation exhibited a linear elimination phase. For example, the following slopes of linear trends for pharmacokinetic variables were obtained.

| Parameter | Slope | 95% CI for slope |
|---|---|---|
| Cmax (pg/ml) | 0.874 | 0.709-1.039 |
| AUCT (pg.h/ml) | 0.978 | 0.877-1.080 |
| AUCI (pg.h/ml) | 0.895 | 0.797-0.993 |
| t½ (h) | -0.012 | -0.025-0.001 |

The mixed effects model indicates a strong linear relationship between logarithm of the pharmacokinetic variables (Cmax, AUCT and AUCI) and logarithm of the dose. The model also indicated that t½ is not linearly related to the dose.

The geometric LS means of AUCT (1487 pg.h/mL for Fentanyl TAIFUN® at 100 µg, and 3085 pg.h/mL for Actiq® at 200 µg) show that 100 µg TAIFUN® is proportionally as bioavailable as the Reference product (Actiq® 200 µg), but the time to reach Tmax is significantly more rapid (2 minutes for Fentanyl TAIFUN® versus one hour for Actiq®). Correspondingly, a 200 µg dose of fentanyl from Fentanyl TAIFUN® (3435 pg.h/mL) is approximately 10% more bioavailable than the Reference Actiq® 200 µg, but the time to reach Tmax is significantly more rapid, i.e., about 1 minute for fentanyl formulated according to the present disclosure as Fentanyl TAIFUN® versus about one hour for Actiq®. The geometric LS means of Cmax also indicated a greater plasma fentanyl peak concentration in 200 µg Fentanyl TAIFUN® compared to reference Actiq® (945 pg/mL vs. 375 pg/mL, respectively).

Geometric LS means were also calculated for the following parameters as a function of dose of fentanyl administered using Fentanyl TAIFUN®, as well as Actiq® (Reference): AUC 0 minutes to 20 minutes (AUC 0-20); AUC 0 minutes to 30 minutes (AUC 0-30); AUC 20 minutes to infinity (AUC 20-I); and AUC 30 minutes to infinity (AUC 30-I). The calculated geometric LS means of AUC 0-20; AUC 0-30; AUC 20-I; and AUC 30-I are shown below in Table 3.

**Table 3**

| Dose | AUC 0-20 (pg/mL.h) | AUC 0-30 (pg/mL.h) | AUC 20-I (pg/mL.h) | AUC 30-I (pg/mL.h) |
|---|---|---|---|---|
| Reference | 17.940 | 51.187 | 3185.396 | 3147.861 |
| 100 µg | 56.031 | 82.830 | 1773.868 | 1742.744 |
| 200 µg | 139.638 | 201.817 | 3707.698 | 3641.816 |
| 400 µg | 222.851 | 326.491 | 5681.169 | 5572.441 |
| 800 µg | 484.178 | 709.193 | 12070.451 | 11833.447 |

The mean (Standard Deviation (SD)) plasma fentanyl concentrations of the subjects who received the study medication are presented below in Table 4.

**Table 4**

| Dose | Summary Statistic | Cmax (pg/mL) | Tmax^{*} (h) | AUCT (pg/mL.h) | AUCI (pg/mL.h) | t 1/2 (h) |
|---|---|---|---|---|---|---|
| Reference | Mean | 401.844 | 1.000 | 3543.802 | 3808.015 | 17.701 |
| | SD | 169.584 | (0.5-4.0) | 2283.951 | 2424.835 | 11.008 |
| 100 µg | Mean | 610.967 | 0.05 | 1726.990 | 2105.954 | 26.523 |
| | SD | 766.821 | 0.0167-1.0) | 857.112 | 1078.973 | 27.837 |
| 200 µg | Mean | 1146.991 | 0.0167 | 3953.080 | 4287.751 | 20.984 |
| | SD | 688.881 | (0.0167-4.0) | 2006.042 | 2166.566 | 15.208 |
| 400 µg | Mean | 1870.416 | 0.0167 | 6286.454 | 6727.110 | 20.583 |
| | SD | 1734.874 | (0.0167-2.0) | 3256.206 | 3627.149 | 13.429 |
| 800 µg | Mean | 3470.846 | 0.050 | 13640.924 | 14142.294 | 16.293 |
| | SD | 3252.686 | (0.0167-2.0) | 6502.546 | 6904.788 | 4.291 |

| | | | | | | |
|---|---|---|---|---|---|---|
| *Median (range) values are presented for Tmax | | | | | | |

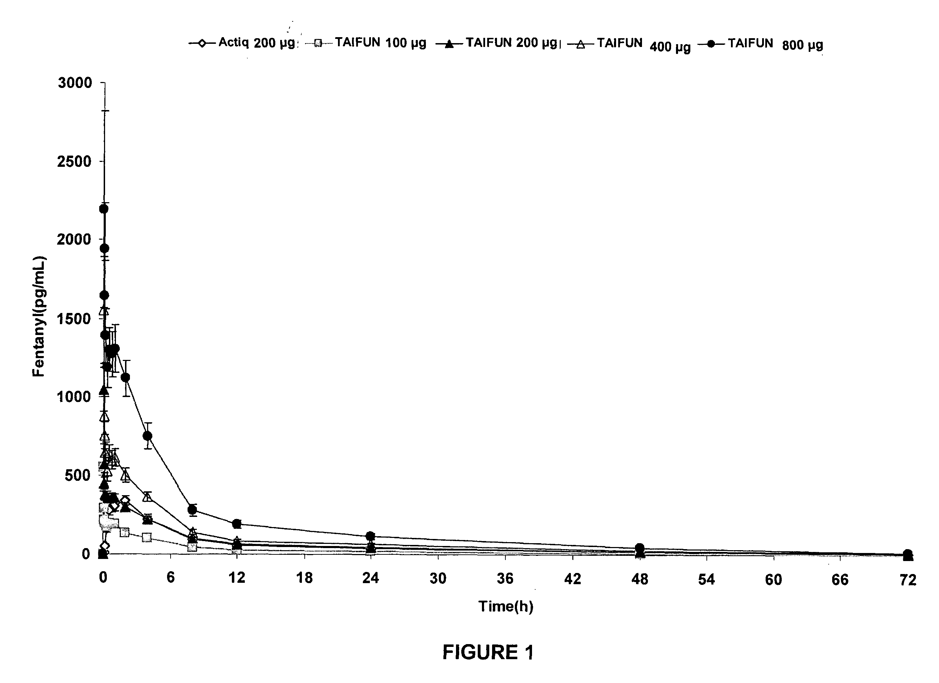

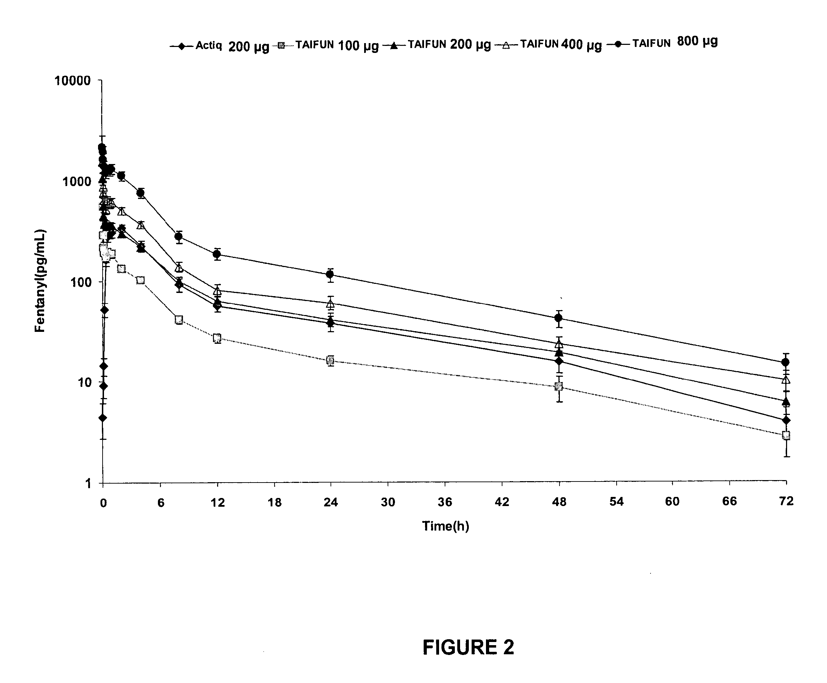

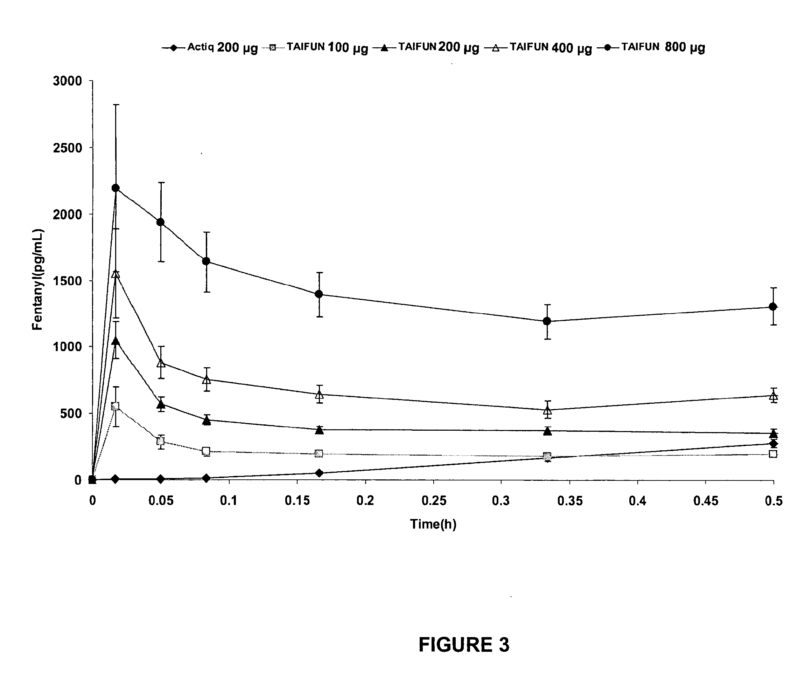

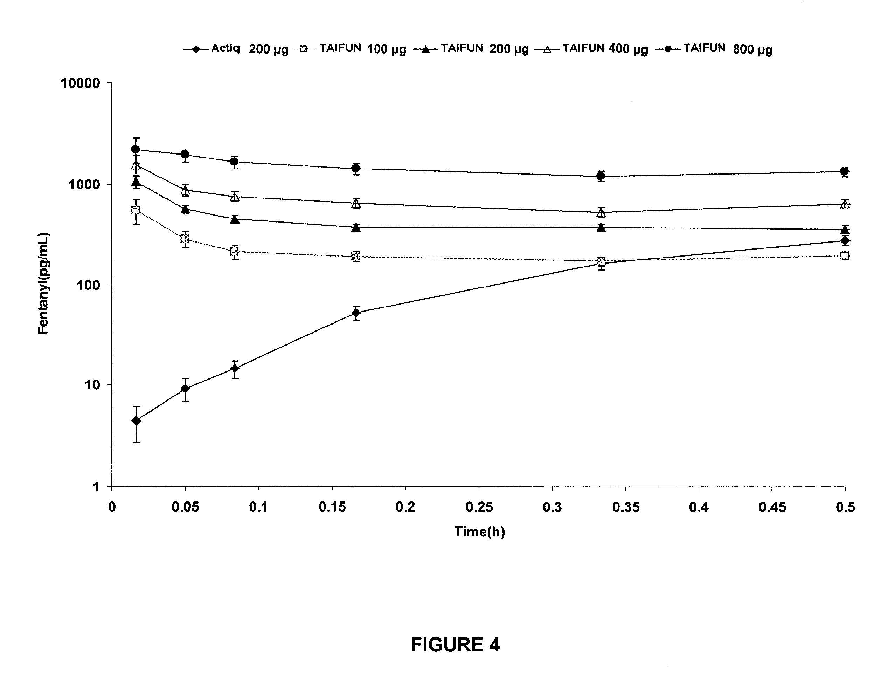

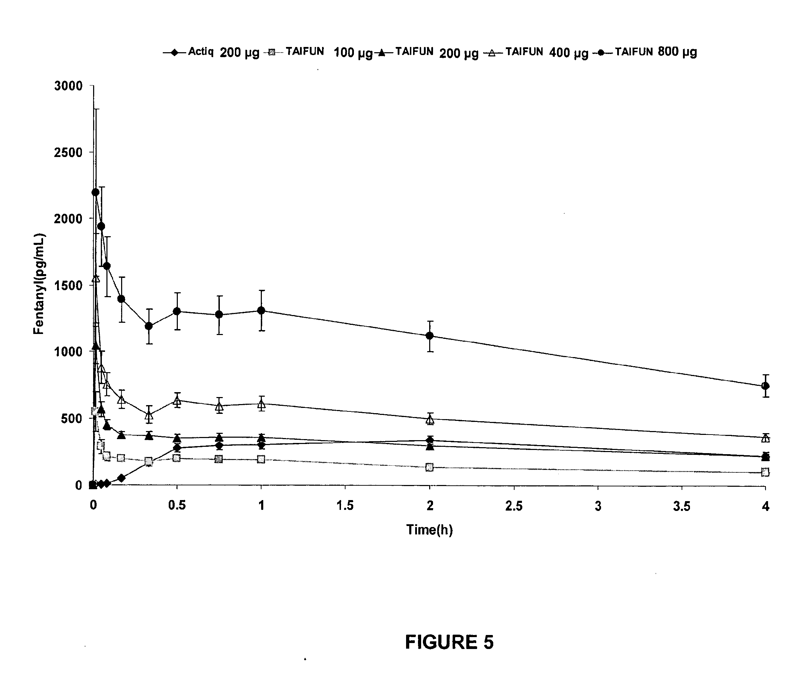

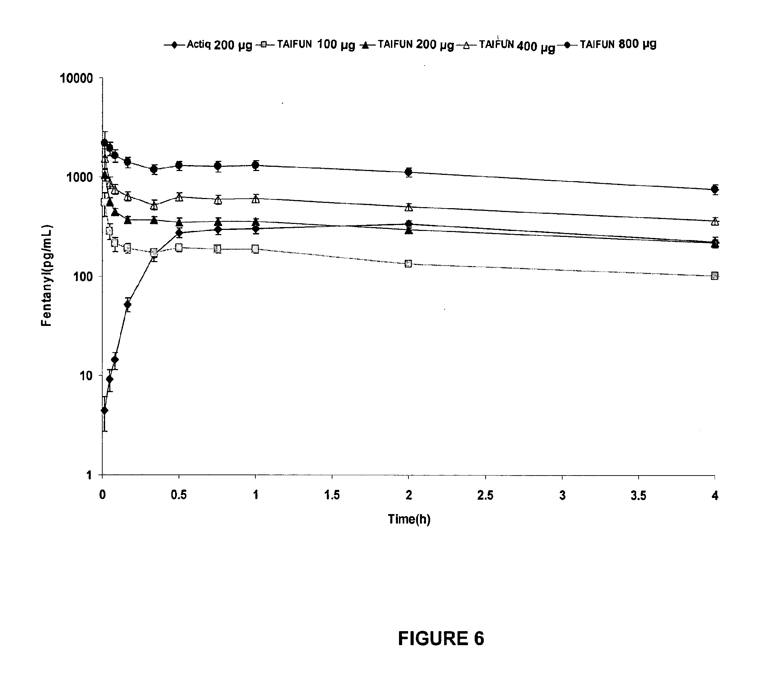

Figures 1-6 shows the mean plasma fentanyl concentrations versus time profiles for 0-72 hours and 0-30 minutes.

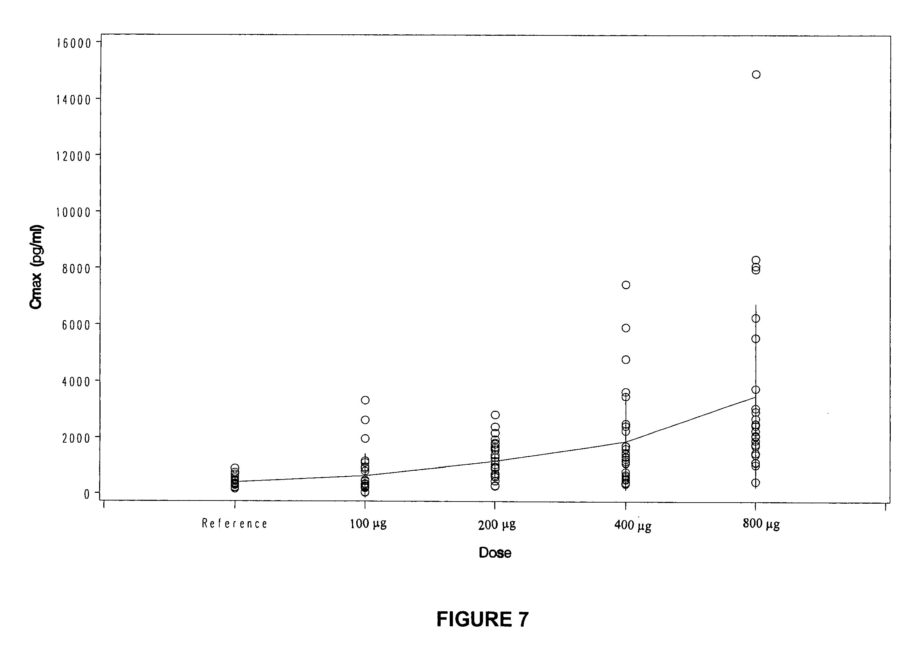

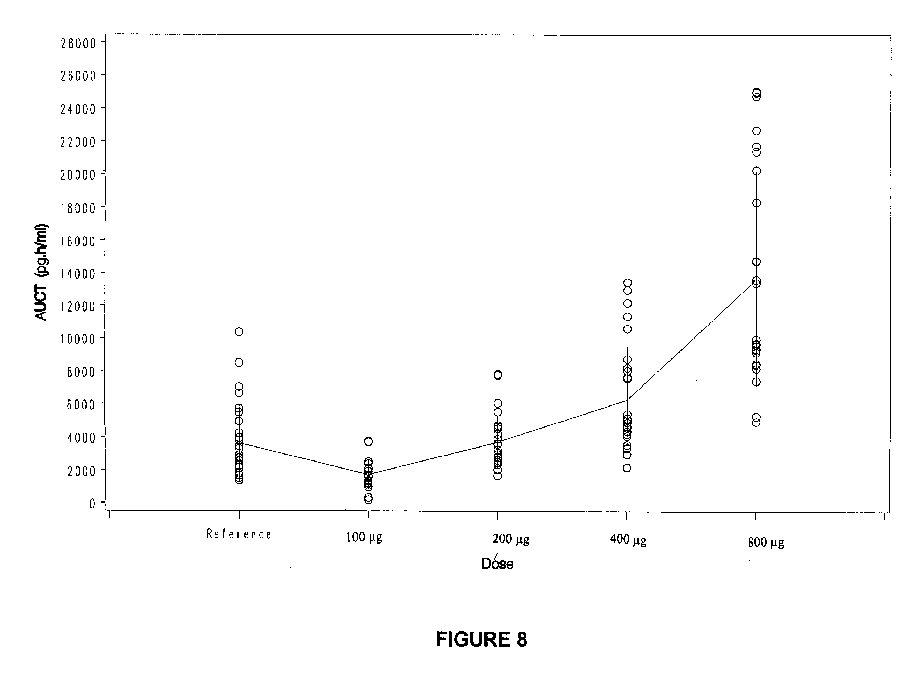

A summary of tests for dose proportionality/independence following the administration of the study Fentanyl TAIFUN® medication is shown in Table 5. Plots of individual Cmax and AUCT values versus dose are shown in summary Figures 7 and 8. The mixed effects model indicates a strong linear relationship between logarithm of the pharmacokinetic variables and logarithm of the dose. The model indicates also that t½ is not linearly related to the dose.

**Table 5**

| Parameter | 100 µg | 200 µg | 400 µg | 800 µg | Slope | 95% CI for Slope |
|---|---|---|---|---|---|---|
| | Geometric LS means | | | | | |
| Cmax (pg/mL) | 362.12 | 945.02 | 1330.99 | 2422.62 | 0.874 | 0.709 - 1.039 |
| AUCT (pg.h/mL) | 1488.72 | 3440.23 | 5578.71 | 12165.63 | 0.978 | 0.877 - 1.080 |
| AUCI (pg.h/mL) | 1845.32 | 3726.52 | 5928.94 | 12531.68 | 0.895 | 0.797 - 0.993 |
| t 1/2 (h) | 26.44 | 21.14 | 20.58 | 16.32 | -0.012 | -0.025 - 0.001 |

A summary of tests for bioavailability within the pharmacokinetic population is shown in Table 6 for 100 µg Fentanyl TAIFUN® and Table 7 for 200 µg Fentanyl TAIFUN®. The Fentanyl TAIFUN® doses of 400 µg and 800 µg were not compared to the Reference medication (Actiq® 200 µg), since the 200 µg Fentanyl TAIFUN® had exceeded the limits of bioequivalence.

**Table 6**

| | 100 µg Fentanyl TAIFUN® | 200 µg Reference | Ratio (%) Test / Reference | 95% C.I.s |
|---|---|---|---|---|
| | Geometric LS means | | | |
| Cmax (pg/mL) | 362.21 | 374.87 | 96.62 | 69.05-135.22 |
| AUCT (pg.h/mL) | 1486.60 | 3084.85 | 48.19 | 39.18 - 59.27 |
| AUCI (pg.h/mL) | 1839.00 | 3318.98 | 55.41 | 45.17 - 67.97 |
| AUC 0-20 (pg.h/mL) | 58.36 | 18.24 | 319.92 | 237.01 - 431.82 |
| AUC 0-30 (pg.h/mL) | 85.61 | 52.15 | 164.18 | 126.26-213.48 |
| AUC 20-I (pg.h/mL) | 1760.97 | 3296.94 | 53.41 | 43.44-65.67 |
| AUC 30-I (pg.h/mL) | 1729.28 | 3258.79 | 53.07 | 43.10-65.33 |
| | Median | | | |
| Tmax (h) | 0.034 | 1.000 | n/a | (p=<0.0001)* |

| | | | | |
|---|---|---|---|---|
| * Wilcoxon Matched Pairs Test | | | | |

**Table 7**

| | 200 µg Fentanyl TAIFUN® | 200 µg Reference | Ratio (%) Test / Reference | 95% C.I.s |
|---|---|---|---|---|
| | Geometric LS means | | | |
| Cmax (pg/mL) | 945.26 | 374.87 | 252.16 | 180.19 - 352.88 |
| AUCT (pg.h/mL) | 3435.35 | 3084.85 | 111.36 | 90.54-136.97 |
| AUCI (pg.h/mL) | 3713.76 | 3318.98 | 111.9 | 91.21-137.27 |
| AUC 0-20 (pg.h/mL) | 139.28 | 18.24 | 763.42 | 565.59-1030.46 |
| AUC 0-30 (pg.h/mL) | 201.45 | 52.15 | 386.32 | 297.10-502.32 |
| AUC 20-I (pg.h/mL) | 3558.57 | 3296.94 | 107.94 | 87.79-132.71 |
| AUC 30-I (pg.h/mL) | 3493.76 | 3258.79 | 107.21 | 87.09-131.99 |
| | Median | | | |
| Tmax (h) | 0.017 | 1.000 | n/a | (p=<0.0001)* |

| | | | | |
|---|---|---|---|---|
| * Wilcoxon Matched Pairs Test | | | | |

A 100 µg dose of fentanyl prepared and administered according to the present disclosure, e.g., as Fentanyl TAIFUN®, provides a pharmacokinetic profile comprising a geometric LS mean Cmax of 362 pg/mL, a median Tmax of 0.034 hours, a geometric LS mean AUCT of 1487 pg.h/mL, a geometric LS mean AUCI of 1839 pg.h/mL, a geometric LS mean AUC 0-20 (AUC 0 minutes to 20 minutes) of 58 pg.h/mL, a geometric LS mean AUC 0-30 (AUC 0 minutes to 30 minutes) of 86 pg.h/mL, a geometric LS mean AUC 20-I (AUC 20 minutes to infinity) of 1761 pg.h/mL, and a geometric LS mean AUC 30-I (AUC 30 minutes to infinity) of 1729 pg.h/mL. A 200 µg dose of fentanyl prepared and administered according to the present disclosure, e.g., as Fentanyl TAIFUN®, provides a pharmacokinetic profile comprising a geometric LS mean Cmax of 945 pg/mL, a median Tmax of 0.0167 hours, a geometric LS mean AUCT of 3435 pg.h/mL, a geometric LS mean AUCI of 3714 pg.h/mL, a geometric LS mean AUC 0-20 of 139 pg.h/mL, a geometric LS mean AUC 0-30 201 pg.h/mL, a geometric LS mean AUC 20-I of 3559 pg.h/mL, and a geometric LS mean AUC 30-I of 3494 pg.h/mL.

A 400 µg dose of fentanyl prepared and administered according to the present disclosure by dosing twice with a 200 µg dose, e.g., as Fentanyl TAIFUN®, provides a pharmacokinetic profile comprising a geometric LS mean Cmax of 1331 pg/mL, a median Tmax of 0.017 hours, a geometric LS mean AUCT of 5579 pg.h/mL, a geometric LS mean AUCI of 5928 pg.h/mL, a geometric LS mean AUC 0-20 of 223 pg.h/mL, a geometric LS mean AUC 0-30 of 326 pg.h/mL, a geometric LS mean AUC 20-1 of 5681 pg.h/mL, and a geometric LS mean AUC 30-1 of 5572 pg.h/mL.

The plasma concentrations of fentanyl formulated according to the present disclosure and administered by inhalation using Fentanyl TAIFUN® increased proportionally to the increasing dose, and t_{1/2} was independent of the dose. Fentanyl TAIFUN® had a substantially faster absorption and higher peak fentanyl concentration (Cmax) than fentanyl from the Reference (Actiq® 200 µg). Median Tmax was between 1 and 3 minutes for Fentanyl TAIFUN®. In contrast, median Tmax for Actiq® 200 µg was 60 minutes. Moreover, there was a greater than 6-fold increase in systemic availability of fentanyl during the first 20 minutes when 200 µg Fentanyl TAIFUN® was compared with Actiq® 200 µg. Thus, Fentanyl TAIFUN® is substantially more bioavailable than the Reference medication during the important first 20-30 minutes after administration. Inhalation of Fentanyl TAIFUN® provides an immediate availability of fentanyl, similar to an intravenous bolus but with lower peak concentration, suggesting the potential of Fentanyl TAIFUN® for rapid pain relief.

Thus, in certain embodiments, the powdered formulation of the present disclosure provides a method to increase in a safe manner the systemic availability of fentanyl during the first 20 minutes after dosing, comprising administering to a patient in need of treatment for breakthrough pain a dose of at least 100 µg of the powdered formulation comprising fentanyl through pulmonary inhalation, preferably formulated as Fentanyl TAIFUN®. In another aspect, the present disclosure provides a method to increase the plasma concentration of fentanyl during a time period of up to two hours or more, as compared with an intravenous injection of fentanyl.

Taken together, Fentanyl TAIFUN® provides a method to combine the desirable properties of both intravenous injection, and transmucosal oral preparations, in being able to produce a very rapid absorption and high early bioavailability (such as with intravenous injection or some conventional inhalation solutions), and to produce a prolonged effective drug concentration (such as with transmucosal oral preparations), but without the undesired properties of the same, i.e. the rapid decrease of the drug concentration and short analgesic action (such as with intravenous injection, or some conventional inhalation solutions), and the slow absorption of the active drug and a slow onset of action ((such as with transmucosal oral preparations).

The key safety parameters examined during this study were (1) Spirometry (FEV₁)/(FVC); (2) Adverse events; (3) Vital Signs (supine blood pressure, pulse, respiratory rate, and pulse oximetry); (4) Electrocardiogram (ECG) parameters (heart rate, PR interval, QRS width, QT interval and QTc interval together with an overall assessment of the ECG as "normal," "abnormal, no clinical significance," or "abnormal, clinically significant"); and (5) Laboratory Safety Screens. Adverse events were coded using the MedDRA drug dictionary. All thirty subjects who were randomized were assessed for adverse events and laboratory test abnormalities. Four subjects were withdrawn due to adverse events during the study. A total of 331 adverse events were reported during the study. Of the 331 adverse events reported during the study, three events were recorded prior to dosing with the study medication. The greatest incidence of adverse events occurred during the dosing of the 400 µg Fentanyl TAIFUN®, with an incidence of 89.3%. The most common adverse events that occurred during the study were nausea, vomiting, fatigue, decreased appetite, headache and somnolence. None of the out-of-normal-range clinical laboratory values were considered to be clinically significant.

During the study, there were no significant changes in physical examinations of the subjects, and the reported adverse events were of similar nature to those following the administration of opioid medications, which are to be expected following the administration of fentanyl. The mean changes from baseline graphs for vital signs showed that there is an increase in supine systolic blood pressure measurements from the 4-hour time point for all fentanyl doses, except for the 100 µg dose, which showed an increase in change from baseline supine pulse rate at the 8-hour time point. An increase in change from baseline for the pulse oximetry was seen from the 1.5-hour time point, but the increase was seen to a greater extent from the 4-hour time point. The mean changes from baseline graphs for spirometry measurements showed an improvement in the FEV₁/FVC values over time. The mean changes from the baseline graphs for ECG measurements showed that there is an increase in the Heart Rate (bpm) measurements from the 8-hour time point. A corresponding decrease in change from baseline QT Interval (mSec) was also seen from the 8-hour time point. The conclusion of the study is that Fentanyl TAIFUN® is considered well-tolerated and safe.

A summary of derived pharmacokinetic data for the fentanyl formulations prepared according to Examples 1 and 2. and administered by pulmonary inhalation in the form of Fentanyl TAIFUN®, is presented in Table 8 below.

**Table 8**

| Dose | Summary Statistic | Cmax (pg/ml) | Tmax (h) | AUCT (pg/ml.h) | AUCI (pg/ml.h) | AUC 0-20 (pg/ml.h) | AUC 0-30 (pg/ml.h) | AUC 20-I (pg/ml.h) | AUC 30-I (pg/ml.h) | t 1/2 (h) |
|---|---|---|---|---|---|---|---|---|---|---|
| Reference | N | 29 | 29 | 29 | 29 | 29 | 29 | 29 | 29 | 29 |
| Reference | Mean | 401.844 | 1.336 | 3543.802 | 3808.015 | 22.740 | 61.110 | 3785.275 | 3746.905 | 17.701 |
| Reference | SD | 169.584 | 0.814 | 2283.951 | 2424.835 | 15.279 | 37.156 | 2413.764 | 2398.081 | 11.008 |
| Reference | CV | 42.202 | 60.889 | 64.449 | 63.677 | 67.190 | 60.802 | 63.767 | 64.002 | 62.192 |
| Reference | Min | 167.820 | 0.500 | 1154.757 | 1113.533 | 4.680 | 12.580 | 1101.390 | 1080.900 | 2.340 |
| Reference | Median | 340.900 | 1.000 | 2762.718 | 2973.784 | 20.180 | 57.270 | 2969.100 | 2954.010 | 16.638 |
| Reference | Max | 892.490 | 4.000 | 10371.106 | 11006.195 | 62.660 | 161.100 | 10943.540 | 10845.310 | 43.997 |
| Reference | Geo. Mean | 372.251 | N/A | 2986.173 | 3206.918 | 17.940 | 51.187 | 3185.396 | 3147.861 | 14.144 |
| Reference | Geo. CV | 40.717 | N/A | 63.434 | 64.287 | 86.188 | 69.875 | 64.486 | 64.902 | 86.408 |
| 100 µg | N | 30 | 30 | 30 | 30 | 30 | 30 | 30 | 30 | 30 |
| 100 µg | Mean | 610.967 | 0.236 | 1726.990 | 2105.954 | 75.572 | 105.977 | 2030.382 | 1999.977 | 26.523 |
| 100 µg | SD | 766.821 | 0.346 | 857.112 | 1078.973 | 59.333 | 73.185 | 1061.597 | 1056.485 | 27.837 |
| 100 µg | CV | 125.509 | 146.609 | 49.630 | 51.234 | 78.512 | 69.058 | 52.286 | 52.825 | 104.956 |
| 100 µg | Min | 34.010 | 0.017 | 196.506 | 352.142 | 7.360 | 10.250 | 343.600 | 338.090 | 2.454 |
| 100 µg | Median | 278.545 | 0.050 | 1584.523 | 1855.059 | 61.930 | 89.825 | 1789.785 | 1766.390 | 14.826 |
| 100 µg | Max | 3310.850 | 1.000 | 3749.511 | 4363.758 | 263.430 | 344.390 | 4221.360 | 4176.180 | 93.013 |
| 100 µg | Geo. Mean | 350.455 | N/A | 1496.718 | 1849.484 | 56.031 | 82.830 | 1773.868 | 1742.744 | 15.766 |
| 100 µg | Geo. CV | 146.838 | N/A | 67.297 | 58.155 | 103.583 | 93.005 | 59.327 | 59.928 | 143.168 |
| 200 µg | N | 29 | 29 | 29 | 29 | 29 | 29 | 29 | 29 | 29 |
| 200 µg | Mean | 1146.991 | 0.258 | 3953.080 | 4287.751 | 155.110 | 219.109 | 4132.640 | 4068.641 | 20.984 |
| 200 µg | SD | 688.881 | 0.760 | 2006.042 | 2166.566 | 62.887 | 81.487 | 2140.301 | 2130.142 | 15.208 |
| 200 µg | CV | 60.060 | 294.337 | 50.746 | 50.529 | 40.544 | 37.190 | 51.790 | 52.355 | 72.477 |
| 200 µg | Min | 272.740 | 0.017 | 1684.820 | 1730.730 | 27.550 | 57.850 | 1685.640 | 1651.630 | 4.238 |
| 200 µg | Median | 963.140 | 0.017 | 3245.772 | 3725.869 | 161.100 | 225.620 | 3559.420 | 3474.590 | 19.105 |
| 200 µg | Max | 2808.990 | 4.000 | 10490.936 | 10770.438 | 284.560 | 369.800 | 10607.750 | 10542.780 | 80.562 |
| 200 µg | Geo. Mean | 937.231 | N/A | 3575.077 | 3865.427 | 139.638 | 201.817 | 3707.698 | 3641.816 | 17.216 |
| 200 µg | Geo. CV | 77.216 | N/A | 46.086 | 47.366 | 55.339 | 46.533 | 48.541 | 49.129 | 70.271 |
| 400 µg | N | 28 | 28 | 28 | 28 | 28 | 28 | 28 | 28 | 28 |
| 400 µg | Mean | 1870.416 | 0.181 | 6286.454 | 6727.110 | 261.031 | 369.337 | 6466.080 | 6357.774 | 20.583 |
| 400 µg | SD | 1734.874 | 0.406 | 3256.206 | 3627.149 | 142.008 | 177.981 | 3553.235 | 3528.353 | 13.429 |
| 400 µg | CV | 92.753 | 224.192 | 51.797 | 53.918 | 54.403 | 48.189 | 54.952 | 55.497 | 65.246 |
| 400 µg | Min | 356.170 | 0.017 | 2191.523 | 2109.464 | 66.100 | 121.480 | 2043.360 | 1971.910 | 4.232 |
| 400 µg | Median | 1324.790 | 0.017 | 4979.344 | 5288.656 | 228.975 | 332.760 | 5054.245 | 4932.030 | 16.968 |
| 400 µg | Max | 7428.720 | 2.000 | 13451.227 | 15238.876 | 591.130 | 766.380 | 14860.240 | 14702.940 | 73.901 |
| 400 µg | Geo. Mean | 1330.989 | N/A | 5578.709 | 5928.944 | 222.851 | 326.491 | 5681.169 | 5572.441 | 17.742 |
| 400 µg | Geo. CV | 99.575 | N/A | 52.294 | 53.741 | 66.196 | 56.520 | 54.237 | 54.807 | 58.241 |
| 800 µg | N | 26 | 26 | 26 | 26 | 26 | 26 | 26 | 26 | 26 |
| 800 µg | Mean | 3470.846 | 0.264 | 13640.924 | 14142.294 | 563.607 | 802.752 | 13578.686 | 13339.542 | 16.293 |
| 800 µg | SD | 3252.686 | 0.441 | 6502.546 | 6904.788 | 308.626 | 392.535 | 6773.916 | 6718.929 | 4.291 |
| 800 µg | CV | 93.715 | 167.430 | 47.669 | 48.824 | 54.759 | 48.899 | 49.886 | 50.369 | 26.336 |
| 800 µg | Min | 438.380 | 0.017 | 4959.487 | 5262.073 | 115.490 | 175.920 | 5080.180 | 4971.210 | 9.552 |
| 800 µg | Median | 2336.225 | 0.050 | 9831.821 | 10025.722 | 482.190 | 677.545 | 9570.220 | 9369.290 | 16.091 |
| 800 µg | Max | 14881.630 | 2.000 | 25025.363 | 26809.115 | 1323.580 | 1705.800 | 25951.260 | 25614.790 | 28.723 |
| 800 µg | Geo. Mean | 2519.753 | N/A | 12228.124 | 12628.054 | 484.178 | 709.193 | 12070.451 | 11833.447 | 15.794 |
| 800 µg | Geo. CV | 93.734 | N/A | 50.858 | 51.658 | 64.420 | 57.157 | 52.699 | 53.174 | 25.556 |

### Example 7

In order to evaluate the fentanyl formulations disclosed herein using Fentanyl TAIfUN® for the management of breakthrough cancer pain in patients, the presently disclosed fentanyl formulations were compared with historical ranges of fentanyl titration in breakthrough pain in cancer patients using orotransmucosal administration of fentanyl. Breakthrough pain, such as pain caused by a malignancy, can be considered to be a transient increase in the amount of moderate-to-severe pain above the levels of persistent pain occurring, for example in cancer patients. Generally the persistent pain in such patients is otherwise muted or controlled with maintenance doses of one or more opioid medications. Such maintenance doses can be, for example, at least 60 mg morphine/day, 50 µg of transdermal fentanyl/hour, or an equianalgesic dose of another opioid for a week or longer. The dosage level of fentanyl useful to treat breakthrough pain, for example in a cancer patient, may not be predicted from the daily maintenance dose of opioid used to manage the persistent pain in the patient. The dosage level of fentanyl useful to treat breakthrough pain is preferably determined by dose titration to a dosage level which provides an individual patient with an adequate analgesic effect while minimizing potential side effects, such as respiratory depression or hypoventilation.

A group of patients who were suffering from periodic episodes of breakthrough pain were titrated to an efficacious analgesic dose, using fentanyl formulated as disclosed in Examples 1 and 2 for inhalation administration as Fentanyl TAIFUN®, starting from the lowest dose of 100 µg. A comparison between the successful rates of titration at each dose level using fentanyl formulated as disclosed herein for inhalation administration as Fentanyl TAIFUN® is shown below in Table 9, with a comparison to published information of titration success of oral transmucosal formulations of fentanyl (Fentora™ and Actiq®).

**Table 9**

| Dose (µg) | Taifun (23 patients) | Fentora™ (80 patients) | Actiq (92 patients) |
|---|---|---|---|
| 100 | 39% | 10% | |
| 200 | 74% | 20% | 10% |
| 400 | 96% | 36% | 25% |
| 600 | | 45% | 36% |
| 800 | | 65% | 50% |
| 1200 | | | 60% |
| 1600 | | | 71% |

As shown in Table 9, the fentanyl formulations disclosed herein through inhalation administration are able to treat a surprisingly high percentage of patients suffering from breakthrough pain, particularly from breakthrough pain related to disease progression in cancer, at lower doses as compared to oral transmucosal formulations of fentanyl. Preferably, the dry powdered formulations of fentanyl disclosed herein are administered from a dry powder inhaler such as a TAIFUN® inhaler. At a dose level of 100 µg of fentanyl, breakthrough pain was successfully treated in 39% of individuals in a population of 23 patients who were administered the dry powdered formulation of fentanyl citrate of the present disclosure by inhalation using a TAIFUN® inhaler. When the dose level was raised to 200 µg of fentanyl, breakthrough pain was successfully treated in 74% of the individuals. While a dose level of 400 µg of fentanyl treated the breakthrough pain in 96% of individuals.

Thus, successful titration of breakthrough pain was achieved in 96% of individuals in a population of patients who received between 100 µg equivalents to 400 µg equivalents (4-fold titration range) of fentanyl formulated as the dry powdered formulation of fentanyl citrate of the present disclosure, when administered to each patient of the population by inhalation. In contrast, published studies using transmucosal delivery of fentanyl achieved successful titration of breakthrough pain in only 65% of individuals in a population of patients who received between 100 and 800 µg (8-fold titration range) of fentanyl formulated as Fentora™ for transmucosal administration. In addition, only 71 % of individuals in a population of patients were successfully titrated for breakthrough pain when treated with between 200 µg and 1600 µg (8-fold titration range) of fentanyl formulated as Actiq® for transmucosal administration (Cephalon Inc., see Actiq® Label, approved by the FDA on February 7, 2007, Part 14).

Fentanyl formulated as the dry powdered formulation of fentanyl citrate as disclosed herein, in which the dry powdered formulation was administered by inhalation using a TAIFUN® inhaler, was efficacious in treating severe pain within a narrow range of dose titration (4-fold titration range), and at doses that were low (100-400 µg of fentanyl) and well tolerated. In comparison, in two published studies with transmucosal formulations of fentanyl (Fentora™ and Actiq®), which are the only comparable published studies of fentanyl administration in breakthrough pain, a broader titration range (8-fold titration range) was required, and a lower rate of efficacious treatment was observed. Thus the formulations of the present disclosure have important advantages over these commercially available fentanyl treatments for breakthrough pain.

## Claims

1. A method for the preparation of a physically stable and homogenous powdered pharmaceutical composition for pulmonary administration comprising (1) particles comprising an analgesic, and (2) particles comprising a pharmaceutically acceptable carrier for pulmonary inhalation, the method comprising:
(a) suspending the particles comprising the analgesic and the pharmaceutically acceptable carrier in a suspending solvent, thereby forming a suspension, and
(b) removing the suspending solvent from the suspension of step (a) to obtain the pharmaceutical composition.

2. The method of claim 1, wherein the particles are essentially insoluble, or sparingly soluble, or slightly soluble, or partially soluble in the suspending solvent.

3. The method of claim 1 or 2, wherein the powdered pharmaceutical composition is a dry powdered pharmaceutical composition.

4. The method of any one of claims 1 to 3, wherein the analgesic is an opioid.

5. The method of claim 4, wherein the opioid is fentanyl or a pharmaceutically acceptable salt thereof.

6. The method of claim 4 or 5, wherein the opioid is fentanyl citrate.

7. The method of claim 6, wherein the particles comprising fentanyl citrate have a mean particle size of about 1 to about 5 microns.

8. The method of any one of claims 1 to 7, wherein the pharmaceutically acceptable carrier is selected form the group consisting of lactose, lactose monohydrate, glucose, mannitol, and combinations thereof.

9. The method of any one of claims 1 to 8, wherein the particles comprising the pharmaceutically acceptable carrier have a mean particle size of about 50 to about 130 microns.

10. The method of any one of claims 1 to 9, wherein the suspending solvent comprises a combination of a non-polar hydrocarbon and a semi-polar hydrocarbon.

11. The method of claim 10, wherein the semi-polar hydrocarbon has a dielectric constant of equal to or greater than about 5 and less than about 50.

12. The method of any one of claims 1 to 9, wherein the suspending solvent comprises a hydrocarbon or a combination of hydrocarbons capable of forming an azeotrope with water.

13. The method of claim 12, wherein the hydrocarbon capable of forming an azeotrope with water is selected from the group consisting of pentane, hexane, heptane, and combinations thereof.

14. The method of claim 12 or 13, wherein the hydrocarbon capable of forming an azeotrope with water is n-hexane.

15. The method of any one of claims 12 to 14, wherein one of the hydrocarbons is an alcohol.

16. The method of any one of claims 12 to 15, wherein one of the hydrocarbons is 2-butanol.

17. The method of any one of claims 1 to 16, wherein the suspending solvent comprises a combination of n-hexane and 2-butanol.

18. The method of any one of claims 1 to 17, wherein the suspending solvent is removed by evaporation, or filtration, or spray drying, or centrifugation, or any combination thereof.

19. The method of any one of claims 1 to 18, further comprising applying ultrasound, mechanical agitation, or a combination thereof to triturate and deagglomerate the suspended particles.

20. A physically stable and homogenous powdered pharmaceutical composition for pulmonary administration comprising (1) particles comprising an analgesic, and (2) particles comprising a pharmaceutically acceptable carrier for pulmonary inhalation, obtainable by a method according to any one of claims 1 to 19.

21. The composition of claim 20, wherein the composition has a fine particle fraction of between 10% and 60%.

22. A pharmaceutical composition for pulmonary administration to human patients, comprising from about 100 ìg to about 800 ig of fentanyl or a pharmaceutically acceptable salt thereof, said composition providing a mean peak concentration of fentanyl from a mean of about 30 seconds to about 10 minutes after administration, and a concentration of fentanyl that does not decrease more than about 40% between about 10 minutes and about 2 hours after administration.

23. The composition of claim 22, providing a mean peak concentration of fentanyl from a mean of about 30 seconds to about 10 minutes after administration, and a concentration of fentanyl that does not decrease more than about 20% between about 10 minutes and about 1 hour after administration.

24. The composition of claim 22, wherein the pharmaceutical composition is a dry powdered pharmaceutical composition.

25. The composition of claim 22 to 24, wherein the mean peak concentration of fentanyl is from about 300 pg/mL to about 3000 pg/mL of fentanyl.

26. The composition of any one of claims 22 to 25, wherein between about 10 minutes and about 2 hours after administration the mean maximum plasma concentration of fentanyl is about 180 pg/mL to about 1800 pg/mL.

27. A physically stable and homogenous powdered pharmaceutical composition for pulmonary administration comprising fentanyl or a pharmaceutically acceptable salt thereof, and a pharmaceutically acceptable carrier for pulmonary inhalation, wherein the composition has a fine particle fraction of between 10 and 60%.

28. An inhalation device comprising the pharmaceutical composition of any one of claims 20 to 27, wherein the inhalation device is designed to repeatedly meter a single dose quantity of the composition.

29. The device of claim 28, wherein the inhalation device comprises the pharmaceutical composition in a single dose quantity, wherein the single dose quantity comprises 100 ìg, 200 ìg, 400 ìg, or 800 ìg of fentanyl or a pharmaceutically acceptable salt thereof.

30. Use of the composition of any one of claims 20 to 27 for the preparation of a medicament for treating pain in human patients.

31. A pharmaceutical composition for pulmonary administration to human patients, comprising an analgesic, said composition providing a mean peak concentration of the analgesic from a mean of about 30 seconds to about 10 minutes after administration, and a concentration of the analgesic that does not decrease more than about 20% between about 10 minutes and about 1 hour after administration.

32. The composition of claim 31, providing a mean peak concentration of the analgesic from a mean of about 30 seconds to about 10 minutes after administration, and a concentration of the analgesic that does not decrease more than about 40% between about 10 minutes and about 2 hours after administration.
